# EUROPEAN PATENT SPECIFICATION

(11) **EP 4 149 347 B1**
(45) Date of publication and mention of the grant of the patent: **05.02.2025**
(21) Application number: 21730011.0
(22) Date of filing: 10.05.2021
(51) Int. Cl.: A61B 5/00, H04W 4/80

(54) **SYSTEM FOR TRANSMISSION OF SENSOR DATA USING DUAL COMMUNICATION PROTOCOL**
SYSTEM ZUR ÜBERTRAGUNG VON SENSORDATEN MIT EINEM DUALEN KOMMUNIKATIONSPROTOKOLL
SYSTÈME POUR LA TRANSMISSION DE DATA SENSORIEL UTILISANT UN DOUBLE PROTOCOLE DE COMMUNICATION

(30) Priority: 12.05.2020 US 202063023711 P; 07.08.2020 US 202063062939 P
(43) Date of publication of application: 22.03.2023
(73) Proprietor: Masimo Corporation, Irvine, CA 92618 (US)
(72) Inventor: AL-ALI, Ammar, Irvine, California 92618 (US); SCRUGGS, Stephen, Irvine, California 92618 (US); PRIDDELL, Richard, Irvine, California 92618 (US); DE JONG, Chad A., Irvine, California 92618 (US); KINAST, Eric Karl, Irvine, California 92618 (US); HWANG, Jung Soo, Irvine, California 92618 (US); HANG, Steven, Irvine, California 92618 (US)
(74) Representative: Michalski Hüttermann & Partner Patentanwälte mbB
(86) International application number: PCT/US2021/031625
(87) International publication number: WO 2021/231326

(56) References cited:
- WO-A1-2012/112891
- US-A1- 2020 138 288
- US-B2- 9 443 059

## Description

### FIELD OF THE DISCLOSURE

The present disclosure relates to physiological sensors and wireless pairing devices. More specifically, the present disclosure relates to collection of physiological data using physiological sensors and transmitting the data to nearby computing systems using a wireless pairing device.

### BACKGROUND

Conventional physiological measurement systems are limited by the patient cable connection between sensor and monitor. A patient must be located in the immediate vicinity of the monitor. Also, patient relocation requires either disconnection of monitoring equipment and a corresponding loss of measurements or an awkward simultaneous movement of patient equipment and cables. Various devices have been proposed or implemented to provide wireless communication links between sensors and monitors, freeing patients from the patient cable tether.

US 2020/138288 A1 describes medical patient monitoring sensor devices including a disposable sensor assembly and a reusable pairing device.

WO 2012/112891 A1 describes a physiological probe that comfortably attaches to the base of the patient's thumb, thereby freeing up their fingers for conventional activities in a hospital, such as reading and eating.

US 9443059 B2 describes systems and methods of evaluating an association between a wireless sensor and a monitored patient which include a plurality of peripheral electronic devices each having a wireless communication system and a sensor.

### SUMMARY

This disclosure describes, among other things, embodiments of systems, devices, and methods for collecting patient physiological data and transmitting the data to nearby computing systems via wireless transmission.

The invention is defined by the features of the independent claim.

According to one aspect of the present disclosure, not claimed, a system for collecting physiological data from a patient is disclosed
The system can include a disposable module and a reusable module. The disposable module can include a sensor element that can collect physiological data from a patient, a memory, and a battery. The reusable module can include a processor, a memory, and a wireless communication module that can establish a wireless communication with a patient monitoring system. The memory of the reusable module can store the physiological data prior to the wireless communication module establishing the wireless communication. The processor of the reusable module can receive the physiological data from the sensor element of the disposable module when the reusable module is coupled with the disposable module.

The system can include one or more of following features: the disposable module can include a dock coupled to the attachment mechanism and a housing. The housing can house the memory and the battery. The sensor element can be housed within the housing. The sensor element can be coupled to the housing via a cable assembly. The processor of the reusable module can transmit sensor signal to the sensor element of the disposable module. The sensor signal can cause the sensor element to collect the physiological data from the patient. The wireless communication module can establish the wireless communication with the patient monitoring system when the wireless communication module is within a predetermined distance from the patient monitoring system. The wireless communication module can transmit an identification information to the patient monitoring system when the wireless communication module is within the predetermined distance from the patient monitoring system. The patient monitoring system, upon receiving the identification information from the wireless communication module, can create association with the wireless communication module. The identification information can include an identifier that uniquely identifies the disposable module. The patient monitoring system can use the identifier to establish the wireless communication with the reusable module. The disposable module can include an attachment mechanism, wherein the attachment mechanism can couple the disposable module to the patient. The attachment mechanism can be a hospital band. The attachment mechanism can include a radio frequency identifier. The battery of the disposable module can provide power for the reusable module when the disposable module is coupled with the reusable module. The memory of the reusable module can store the physiological data between about 6 hours and about 30 days. The memory of the reusable module may store the physiological data for a length of time prior to establishing or detecting wireless communication. The length of time may be provided by a user and may be user configurable. In some cases, the user may not provide the length of time for storing the physiological data within the memory of the reusable module. The memory of the reusable module can store the physiological data for a default length of time, for example, prior to the wireless communication module of the reusable module establishing wireless communication. The default length of time may be stored within the memory of the reusable module. The physiological data can be collected and stored in the memory of the disposable module when irregularities are sensed. The irregularities can include at least one of: low blood pressure readings, high blood pressure readings, low respiratory rate readings, high respiratory rate readings, blood oxygen desaturations, irregular heartbeats, consistently low or declining blood oxygen saturation readings, low heart rates, or high heart rates. The processor of the reusable module can transmit the physiological data to a local or a remote storage when a wireless communication between the wireless communication module and an online server is established. The transmission of the stored physiological data can occur automatically or manually. The physiological data collected by the sensor element can have high fidelity. The physiological data collected by the sensor element can have low fidelity. The fidelity of the physiological data stored in the memory can vary. The fidelity of the stored physiological data can vary based at least in part on a length of time specified for storing the physiological data within the memory of the reusable module. The fidelity of the stored physiological data can vary based at least in part a type of physiological data or a type of health-related events. The fidelity of the physiological data collected by the sensor element can vary. The fidelity of the physiological data collected by the sensor element can vary based at least in part on a length of time specified for storing the physiological data within the memory of the reusable module. The fidelity of the stored physiological data collected by the sensor element can vary based at least in part a type of physiological data or a type of health-related events. The physiological data stored in the memory of the reusable module may be downloaded when the battery of the disposable module is depleted. The memory can store the physiological data collected by the sensor element from time reusable module is attached to the disposable module until time the reusable portion is detached from the disposable module or the battery of the disposable module fails.

According to another aspect of the present disclosure, not claimed, a method for collecting physiological data from a patient using a reusable module that can couple with a disposable module including a non-invasive sensor element is disclosed. The method can include detecting a coupling between a reusable module and a disposable module. The method can further include collecting physiological data from the disposable module, wherein the physiological data is collected via a sensor element of the disposable module, and wherein the physiological data is stored within the memory of the reusable module. The method can further include establishing a wireless communication with a remote computing device. The method can further include transmitting the physiological data to the remote computing device via the wireless communication.

The method can include one or more of following features: the physiological data can be stored within the memory of the reusable module for a length of time prior to establishing the wireless communication, wherein the length of time can range between about 6 hours to about 30 days. The length of time can be configurable via a configuration provided by a care provider. The memory can store a default length of time and, when the length of time is not provided, the physiological data can be stored within the memory of the disposable module for the default length of time prior to the wireless communication established between the reusable module and the remote computing device. The physiological data can include health-related events related to the patient. The physiological data can be collected and stored when irregularities are sensed. The irregularities can include at least one of: low blood pressure readings, high blood pressure readings, low respiration rate readings, high respiration rate readings, blood oxygen desaturations, irregular heartbeats, consistently low or declining blood oxygen saturation readings, low heart rates, or high heart rates. The physiological data can be transmitted to the remote computing device when the wireless communication is established. Fidelity of the physiological data can vary at least in part on a length of time specified for storing the physiological data within the memory of the reusable module. The fidelity of the physiological data can vary based at least in part a type of physiological data or a type of health-related events. The physiological data stored in the memory of the reusable module may be downloaded when the battery of the disposable module is depleted.

According to an aspect of the present disclosure, a system for collecting physiological data from a patient according to claim 1 is disclosed.

The system can include one or more of following features: The operation data can include sensor type information associated with the disposable module. The sensor type information can indicate one or more types of sensors associated with the disposable module. The reusable module assembly can be associated with a sensor type, and wherein the disposable module can be validated based at least in part on a comparison between the sensor type associated with the reusable module assembly and the sensor type information associated with the disposable module. Sensor life expectancy can be determined based at least in part on the operation data and sensor life data, and wherein the sensor life expectancy can represent the expected operation time of the disposable module. The sensor life data can include sensor use information and one or more functions, and wherein sensor life data can be stored in the memory of the disposable module. The physiological data can be stored in the first memory for a length of time. The length of time can range between about 6 hours to about 30 days. The length of time can be configurable via a configuration provided by a care provider. The first memory can store a default length of time, and wherein the first memory can store the physiological data for the default length of time prior to the wireless communication module establishing the wireless communication when the length of time is not provided. The physiological data can include health-related events related to the patient. The physiological data can be stored when irregularities are sensed. The irregularities may include at least one of: low blood pressure readings, high blood pressure readings, low respiratory rate readings, high respiratory rate readings, blood oxygen desaturations, irregular heartbeats, consistently low or declining blood oxygen saturation readings, low heart rates, or high heart rates. The processor of the reusable module can transmit the stored physiological data to a local or a remote storage via the wireless communication module when a wireless communication between the wireless communication module and an online server is established. The transmission of the stored physiological data can occur automatically or manually. The physiological data collected by the sensor element can have high fidelity. The physiological data collected by the sensor element can have low fidelity. Fidelity of the physiological data stored in the memory can vary. The fidelity of the stored physiological data can vary based at least in part on the predetermined length of time. The fidelity of the stored physiological data can vary based at least in part a type of physiological data or a type of health-related events. Fidelity of the physiological data collected by the sensor element can vary. The fidelity of the physiological data collected by the sensor element can vary based at least in part on the predetermined length of time. The fidelity of the stored physiological data collected by the sensor element can vary based at least in part a type of physiological data or a type of health-related events. The memory can store the physiological data collected by the sensor element from time reusable module is attached to the disposable module until time the reusable portion is detached from the disposable module or the battery of the disposable module fails.

According to another aspect of the present disclosure not claimed, a method of validating a disposable module is disclosed. The method can include detecting a coupling between a disposable module and a reusable module. The method can further include accessing operation data associated with the disposable module. The method can further include analyzing the operation data. The method can further include, based at least in part on the analysis of the operation data, validating the disposable module.

The method can include one or more of following features: detecting the coupling between the disposable module and the reusable module can include determining that the reusable module is receiving power from the disposable sensor module. The disposable module can include a memory that can store the operation data. Analyzing the operation data can include identifying sensor type information form the operation data. comparing the sensor type information with a sensor type associated with the reusable module, and based at least in part on the comparison between the sensor type information with a sensor type associated with the reusable module, determining that the disposable module is compatible with the reusable transmitter module.

For purposes of summarizing the disclosure, certain aspects, advantages, and novel features have been described herein. Of course, it is to be understood that not necessarily all such aspects, advantages, or features will be embodied in any particular embodiment.

### BRIEF DESCRIPTION OF THE DRAWINGS

FIG. 1 illustrates an embodiment of a sensor system including sensors attached to a patient and transmitting patient physiological data to a computing device via cable.
FIG. 2A illustrates another embodiment of a sensor system including sensor assemblies collecting and wirelessly transmitting patient physiological data to a computing device.
FIG. 2B illustrates a schematic diagram of an embodiment of a sensor assembly and a computing device, showing additional details of the sensor assembly.
FIG. 2C illustrates a wiring diagram of an embodiment of a sensor assembly.
FIG. 3A illustrates a perspective view of an embodiment of a sensor assembly for collecting and wirelessly transmitting patient physiological data to a computing device.
FIG. 3B illustrates an exploded, top perspective view of the sensor assembly of FIG. 3A.
FIG. 3C illustrates an exploded, bottom perspective view of the sensor assembly of FIG. 3A.
FIG. 3D illustrates a top view of an embodiment of a sensor assembly.
FIG. 4 illustrates a perspective view of another embodiment of a sensor assembly for collecting and wirelessly transmitting patient physiological data to a computing device.
FIG. 5 illustrates a perspective view of another embodiment of a sensor assembly for collecting and wirelessly transmitting patient physiological data to a computing device.
FIGs. 6A and 6B illustrate various views of a flex circuit of a disposable module of a sensor assembly.
FIGs. 6C and 6D illustrate sides views of the flex circuit of FIG. 6A, showing a change of a configuration of the flex circuit.
FIGs. 7A-7K illustrate various perspective view of different embodiments of sensor assembly coupled with various embodiments of attachment mechanisms.
FIGs. 8A-8C illustrate various views of a dongle operatively connected to the computing device.
FIGs. 9A-9C illustrate a reusable module and a computing device coupled to a dongle, providing additional details for a method of pairing the reusable module with the computing device.
FIGs. 10A-10D illustrate various perspective views of the reusable module and the disposable module of FIG. 3A attached to a wrist of a patient, showing additional details for a method of mating the reusable module with the disposable module.
FIG. 11A illustrates a method of establishing a wireless communication using a reusable module, a disposable module, and a computing device for acquiring and displaying patient physiological parameters.
FIG. 11B illustrates another method of establishing wireless communication using a reusable module, a disposable module, and a computing device for acquiring and displaying patient physiological parameters.
FIG. 12 illustrates another embodiment of a method of acquiring and displaying patient physiological parameters using a reusable module, a disposable module, and a computing device.
FIG. 13A illustrates a mobile application for establishing a wireless communication with a reusable module.
FIGs. 13B-13E illustrate various views of the mobile application of FIG. 13A displaying patient parameters in various display formats.
FIG. 14A illustrates a block diagram of an embodiment of a memory of a disposable module.
FIG. 14B illustrates a method of identifying a disposable module based at least in part on operation data stored in a memory of the disposable module.
FIGs. 15A and 15B illustrates various embodiments of a backup power device for a sensor assembly.
FIG. 16A illustrates a block diagram of an example sensor assembly and an example patient monitoring device in wireless communication.
FIG. 16B illustrates a schematic diagram showing wireless communications between a sensor assembly, patient monitoring devices, and a network.
FIG. 16C illustrates an example method of transmitting physiological data using a sensor assembly.
FIGs. 16D and 16E illustrate example methods of identifying a patient monitoring device and transmitting patient physiological data to the identified patient monitoring device.
FIG. 17 illustrates a schematic diagram showing an example environment for transmitting patient physiological data from a reusable module to user computing devices.

### DETAILED DESCRIPTION

### Introduction

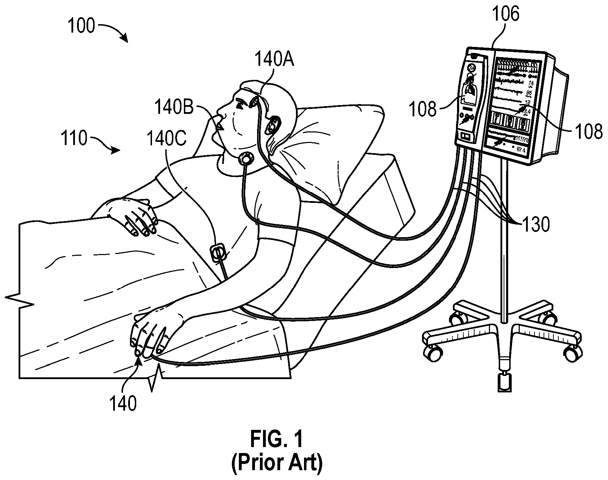

Wired solution for sensors may be cumbersome and difficult to manage when there are multiple sensors attached to a patient as shown in FIG 1. For example, the cable for the sensors can be tangled and damaged after repeated use. Moreover, since the sensors are tethered to a patient health monitor, patients have to be located proximate to the health monitor and movement of the patients can be limited. If a longer cable is required, the sensor and the cable have to be replaced together. Similarly, the sensors being tethered to the monitor can make transportation of the patient very difficult as it would require the patient to remain close to the monitor during transportation or disconnecting the sensors which would result in loss of measurements.

### Overview

FIG. 1 illustrates an example of a sensor system 100 including a computing device 106 coupled sensors 140A, 140B, 140C, 140D via a cable 130, where the sensors are attached to a patient 110. The computing system 106 can include a display 108 that can display various physiological parameters. The sensors 140A, 140B, 140C, 140D can collect various types of physiological data from the patient 110 and transmit the data to the computing system 106 via the cable 130. Some example of the sensors 140A, 140B, 140C, 140D include, but not limited to, a rainbow acoustic monitoring sensor (RAM), O3 Regional Oximetry sensor, SpO2 sensor, a blood pressure sensor, an ECG sensor, and the like.

However, the cables 130 can be cumbersome to the patient and prone to tangling. The cables 130 can develop kinks and be damaged over time. In addition, because the sensors 140A, 140B, 140C, 140D are connected to the computing system 106 via the cables 130, location of the computing system 106 can be restricted to the lengths of the cables 130 attached to the sensors 140A, 140B, 140C, 140D. The cables 130 can also restrict patient movements. Therefore, a wireless solution including wireless communication capacity between the sensors and the computing device may resolve some of the concerns of the wired configuration. The wireless configuration can eliminate the need of the cables 130 between the sensors and the computing device and thus provide greater patient mobility.

However, the wireless solutions may have their own limitations. For example, wireless patient monitoring sensors require internal power source (for example, battery), which can have limited capacity due to size of the sensors. In addition, since continuous data collection and wireless transmission can require significant power usage, operation of the sensors can be very limited. Moreover, it may be expensive to replace the entire device when the internal battery is depleted. Furthermore, having a rechargeable battery may not be suitable in a hospital environment where nurses might not have enough time to wait for the battery to recharge. Also, it may not be ideal for a patient to wait for the battery to recharge in time of need. Accordingly, it can be advantageous to provide a sensor system that is compatible with existing sensors and monitors and is capable of wireless communication as discussed herein.

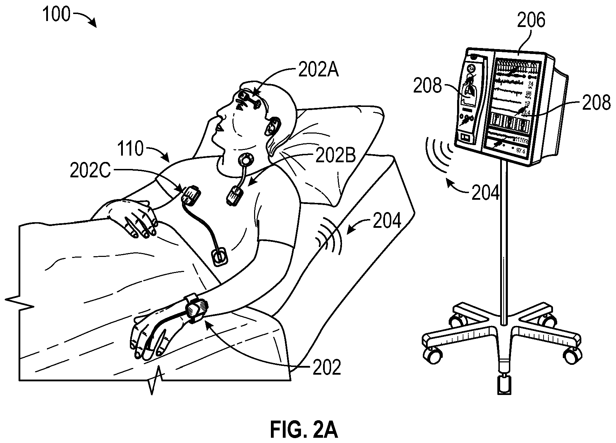

FIG. 2A illustrates the sensor system 100 including a computing device 206 wirelessly receiving patient physiological data of the patient 110 from sensor assemblies 202A, 202B, 202C, 202D. The sensor assemblies 202A, 202B, 202C, 202D can establish communication with the computing device 206 such that data can be wirelessly transmitted between the sensor assemblies 202A, 202B, 202C, 202D and the computing device 206. The computing device 206 can include a display 208 that can display patient parameters determined from the patient physiological data received from the sensor assemblies 202A, 202B, 202C, and 202D.

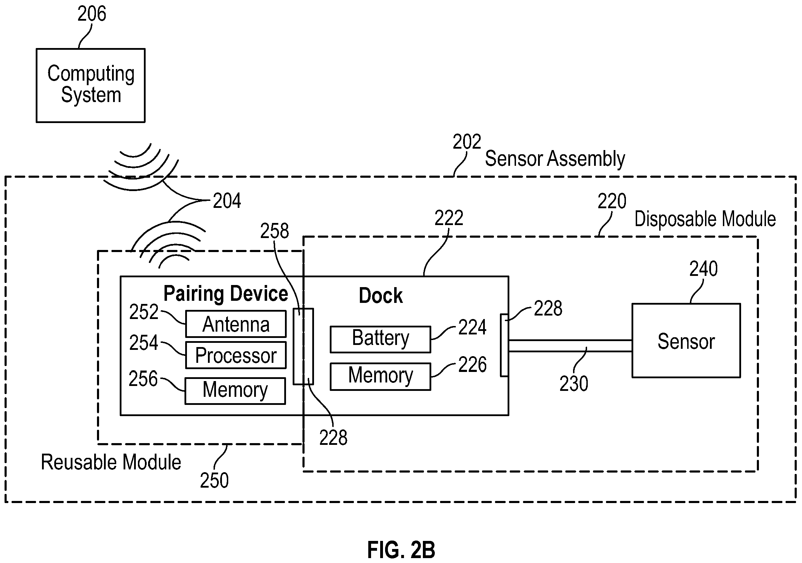

FIG. 2B illustrates a schematic diagram the sensor assembly 202 wirelessly connected to a computing device 206. The sensor assembly 202 can include a disposable module 220 and a reusable module 250. The reusable module 250 can be a pairing device capable of establishing wireless connection with the computing device 206. In some implementations, reusable module 250 is a transmitter device that can transmit to and receive data from nearby computing devices, for example, the computing device 206.

The disposable module 220 can include a dock 222 coupled to a sensor 240 via a cable 230. The dock 222 can be removably connected to the reusable module 250. The reusable module 250 and the computing device 206 can together establish a wireless communication 204 and perform wireless transmission of data between. The reusable module 250 can transmit patient physiological parameters to the computing device 206, where the parameters are calculated from raw physiological data collected by the sensor 240. The transmitted patient data can be raw data collected by the sensor 240.

The reusable module 250 alone or in combination with the dock 222 can perform signal processing on the raw physiological data and transmit the processed physiological data to the computing device 206. The reusable module 250 can establish wireless communication 204 with the computing device 206 to allow data be transmitted between the reusable module 250 and the computing device 206. The reusable module 250 can establish wireless communication 204 with one or more computing devices 206. As shown in FIG. 2A, the computing device 206 can establish wireless communication 204 with the sensor assemblies 202A, 202B, 202C, and 202D. The computing device 206 can establish wireless communication 204 with less than four or more than four sensor assemblies 202.

The reusable module 250 can establish wireless communication 204 with portable mobile devices such as mobile phone, smartphone, tablets, and the like. The computing device 206 can be a hospital patient monitoring system, which includes various types of monitors capable of displaying patient health data. The computing device 206 can be a mobile monitoring system or a personal mobile device. The computing device 206 can be Root^{®} Platform, a patient monitoring and connectivity platform available at Masimo Corporation, Irvine, CA. A mobile physiological parameter monitoring system usable with the cable is described in U.S. Patent No. 9,436,645, issued on September 6, 2016, titled "MEDICAL MONITORING HUB".

The cable 230 can be flexible or non-flexible. The cable 230 can be a thin film including electrical circuitries. The cable 230 can be surrounded by different types of electrical insulating material. The cable 230 can be substantially flat or round.

The sensor 240 can be an acoustic sensor, ECG sensor, EEG sensor, SpO2 sensor, or any other types of patient monitoring sensors. The sensor 240 can include one or more emitters and detectors. The emitters can be low-power, high-brightness LEDs (light-emitting diodes) to increase the life of the batteries 224. The sensor 240 can measure raw physiological data responsive to various types of patient physiological parameters including, but not limited to, temperature, blood pressure, blood oxygen saturation, hemoglobin level, electrocardiogram, and the like. The sensor measurements can be used by physicians to determine patient conditions and treatment for the patient. The sensor 240 can transmit the raw physiological data to the dock 222 via the cable 230. The sensor 240 and the dock 222 may form a unitary body such that the dock 222 receives the physiological data directly from the sensor 240 without the cable 230. The dock 222 can be integrated with one or more of the sensors 340.

The sensor 240 can output a raw sensor signal or a conditioned sensor signal. The sensor 240 can include a signal processor that can process the raw or conditioned sensor signal to derive and calculate physiological parameters associated with the raw or conditioned sensor signal.

The sensor 240 can perform mixed analog and digital pre-processing of an analog sensor signal to generate a digital output signal. As discussed above, the sensor 240 can include a signal processor that can perform digital post-processing of the front-end processor output. The input sensor signal and the output conditioned signal may be either analog or digital. The front-end processing may be purely analog or purely digital. The back-end processing may be purely analog or mixed analog or digital.

The sensor 240 can include an encoder, which translates a digital word or serial bit stream, for example, into a baseband signal. The baseband signal can include the symbol stream that drives the transmit signal modulation, and may be a single signal or multiple related signal components. The encoder can include data compression and redundancy.

The sensor 240 can include a signal processor, an encoder, and a controller. The sensor 240 can utilize emitters 242 and the detectors 244 to generate sensor signals, such as a plethysmograph signal. The signal processor then can use the sensor signal to derive a parameter signal that can include a real time measurement of oxygen saturation and pulse rate. The parameter signal may include other parameters, such as measurements of perfusion index and signal quality. The signal processor can be an MS-5 or MS-7 board available from Masimo Corporation, Irvine, CA. The signal processing step can be performed by the processor 254 of the reusable module 250, as described above.

The dock 222 can be placed on various locations of a patient's body. For example, the dock 222 is placed on the patient's chest. The dock 222 can be placed on other locations on the patient including, but not limited to, torso, back, shoulder, arms, legs, neck, or head. Various means can be used to affix the dock 222 to the patient. For example, the dock 222 is affixed to the patient with an adhesive. In another example, the dock 222 is affixed to the patient with a fastener, such as tape, laid over at least a portion of the dock 222. The dock 222 can be mechanically attachable to at least one strap, which can wrap around the patient.

The reusable module 250 can receive physiological data from the sensor 240 via the dock 222. The reusable module 250 can wirelessly transmit the physiological data to the computing device 206. The reusable module 240 can couple with the dock 222 to establish an electronic communication between the reusable module 250 and the dock 222. The electrical communication between the dock 222 and the reusable module 250 can allow physiological data to be transmitted from the dock 222 to the pairing device 250. The coupling between the reusable module 250 and the dock 222 can be waterproof or shockproof. The disposable module 220 and the reusable module 250 may be shockproof or waterproof. The disposable module 220 and the reusable module 250 can be durable under various types of environments. For example, the reusable module 250 can be fully enclosed, allowing it to be washed, sanitized, and reused.

As shown in FIG. 2B, the dock 222 can include a memory 226 and battery 224. The reusable module 250 can include an antenna 252, a processor 254, and a memory 256. The antenna 252, the processor 254, and the memory 256 can be operatively connected with one another to allow electronic communication or transmission between them.

The antenna 252 can be an RFID (radio-frequency identification) antenna. The antenna 252 can be a Bluetooth^{®} antenna. The reusable module 250 can include one or more antennae 252. In some aspects, the reusable module 250 includes a first antenna and a second antenna, where first antenna is a receiving antenna and the second antenna is a transmitting antenna. The first antenna can be a transmitting antenna and the second antenna can be a receiving antenna. Both the first antenna and the second antenna can both receive data from or transmit data to the computing device 206. The first antenna can be a passive antenna while the second antenna can be an active antenna. The first antenna can be an active antenna while the second antenna can be a passive antenna. An active antenna can include a built-in amplifier that can amplify certain spectrum or frequency of signals. The first antenna can establish an RFID or NFC (near field communication) connection with the computing device 206 while the second antenna can establish a Bluetooth^{®} connection with the computing device 206. In another aspect, both the first and the second antenna are capable of establishing RFID and/or Bluetooth^{®} wireless connection. The process of establishing wireless communication 204 with the computing device 206 and wirelessly transmitting the patient physiological data to the computing device 206 will be further described below in detail.

The memory 256 can be computer hardware integrated circuits that store information for immediate use for a computer (for example, the processor 254). The memory 256 can store the patient physiological data received from the sensor 240. The memory 256 can be volatile memory. For example, the memory 256 is a dynamic random access memory (DRAM) or a static random access memory (SRAM). The memory 256 can be a non-volatile memory. For example, the memory 256 is a flash memory, ROM (read-only memory), PROM (programmable read-only memory), EPROM (erasable programmable read-only memory), and/or EEPROM (electrically erasable programmable read-only memory).

The memory 256 of the reusable module 250 can store patient physiological data received from the sensor 240. The memory 256 can store electronic instructions that, when accessed, prompts the processor 254 to receive patient physiological data from the memory 226 of the dock 222, store the data in the memory 256, retrieve the data from the memory 256, transmit the data to the antenna 252, and use the antenna 252 to wirelessly transmit the data to the computing device 206. One or more of the actions discussed above can be performed simultaneously. For example, the processor 254 of the reusable module 250 can receive patient physiological data from the memory 226 of the dock 222 and simultaneously store the data in the memory 256. In some implementations, the reusable module 250 receives the patient physiological data directly from the sensor 240 without the memory 226 storing the patient physiological data. The memory 226, as described herein, can store other types of data such as operation data and sensor life data.

The memory 256 can store patient physiological data and/or health-related events related to a patient when the sensor assembly 202 is no longer in range with or is otherwise unable to communicate with the computing system 206. The memory 256, as noted above, can have sufficient capacity to store patient health data and/or health-related events. Optionally, the memory 256 can store patient physiological data regardless of whether the reusable module 250 is paired with the computing device 206. Some examples of the health-related events include arrhythmia, low blood pressure, blood oxygen level (SpO2), and the like. Such data and/or health-related events may be accessed via a mobile application on a mobile device (for example, a smartphone, tablet, and the like). The data collected and stored in the memory 256 may be downloaded and/or transferred to local or remote storage. For example, data can be transferred to a cloud server or doctor's office computer system. The transfer of data can automatically or manually occur when wireless communication between the sensor assembly 202 and, for example, an online server or the computing system 206 is established.

Patient data and/or health-related events can be relayed to a device without a display. In such circumstances, the device can have a light source (for example, an LED) that can, for example, blink in different colors or patterns to tell the patients or medical personnel data has been transferred, an error occurred, the data needs to be reviewed, or something else has happened. Different rules can be used to determine when or in what situations can patient physiological information be transmitted from the sensor assembly 202 to other external devices (for example, monitoring devices, mobile devices, and the like).

In some implementations, the memory 256 may only store patient data and/or health-related events related to a patient when the sensor assembly 202 is no longer in range with or is otherwise unable to communicate with the computing system 206. In some implementations, the patient data and/or health-related events may be stored when irregularities are sensed. The irregularities may include, but not limited to, low blood pressure readings, high blood pressure readings, low respiratory rate readings, high respiratory rate readings, blood oxygen desaturations, irregular heartbeats, consistently low or declining blood oxygen saturation readings, low heart rates, high heart rates, and the like. In some implementations, a combination of irregularities or irregular combination of patient status and/or health parameters may cause the sensor assembly 202 to store patient data and/or health-related events. For example, the sensor assembly 202 can store patient data and/or health-related events when high blood pressure readings and low heartbeat rates. In another example, the sensor assembly 202 can store patient data and/or health-related events when patient movement is low and high heart rate or high blood pressure is detected. In yet another example, the sensor assembly 202 can store patient data and/or health-related events when patient movement is low and blood oxygen level is also low. Any suitable combinations of irregularities or abnormal conditions may be used to trigger the sensor assembly 202 to store patient data and/or health-related events.

In some implementations, the memory 256 may only store select health-related event data. Such a configuration may advantageously maximize or increase the life of the battery 224 and/or the memory 256. For example, this data can be as simple as a time stamp when an event or trigger occurred or it can be a snapshot of data taken just before and just after an event or trigger. Events can include physiologically important events such as a heartbeat abnormality or drop in oxygen saturation. The trigger, indicating the start of an event, can cause a window of data to be stored in the memory. For example, the system can continuously hold a window of data for a certain period of time, for example, 5 minutes. When a trigger is detected, data in the window starting several minutes before the event and leading for several minutes the event can be stored in the memory and held until the data is downloaded to another device. Of course, different amounts of time can be stored before and after a trigger, for example, it could be in the range of 1 second to 24 hours.

In some implementations, the memory 256 can store large amount of data, for example days or weeks of data, prior to establishing wireless communication with, for example, the computing system 206. In some implementations, the memory 256 can store up to 96 hours or more of data prior to establishing wireless communication with, for example, the computing system 206. In some implementations, the memory 256 can store up to 30 days of data. The length of time the sensor assembly 202 can collect and store patient data and/or health-related events prior to establishing wireless communication with, for example, the computing system 206, can vary between about 1 hour and about 30 days, between about 3 hours and about 28 days, between about 6 hours and about 21 days, between about 12 hours and about 14 days, between about 24 hours and 7 days, or about 1 hour, about 3 hours, about 6 hours, about 12 hours, about 24 hours, about 72 hours, about 7 days, about 14 days, about 21 days, about 28 days, about 30 days, or ranges between any two of aforementioned values. In this configuration, the device can be worn by a patient at home for a period of monitoring and then downloaded by a doctor at a doctor's office via a wired or wireless connection. The data can also be stored during a period of time when no network connection is detected and then downloaded as soon as a wired or wireless network connection is detected.

In some implementations, users may provide a length of time for which the memory 256 stores patient physiological data prior to establishing or detecting wireless communication. This can be advantageous in non-critical situations in which real-time patient monitoring and management may not be necessary. For example, a care provider may request a patient to revisit a doctor's office in a week and provide a sensor assembly 202 configured to store patient physiological data in the memory 256 for the next seven days. As such, when the patient visits the doctor's office a week later, the care provider can access the data collected and stored in the memory 256 via the reusable module 250. In some implementations, the sensor assembly 202 may be dropped off at the doctor's office, shipped (e.g., via a mail) to the doctor's office. Additionally or alternatively, the data stored in the memory 256 may be automatically or manually uploaded to and stored in a server (e.g., cloud server) to which the care provider (e.g., a doctor) has an access to. In some implementations, the data stored in the memory 256 may be uploaded via, for example, a web interface or a mobile application interface accessible via a user computing device (e.g., a mobile phone, a laptop computer, a desktop computer, a smart phone, a smart device, and the like) as described herein. The sensor assembly 202 may establish wireless communication (e.g., via Bluetooth^{®}) with user computing devices and upload the data stored in the memory 256 to a server accessible to care providers via network communication (e.g., Wi-Fi, 4G, 4G LTE, 5G, 5G LTE, ZigBee, and the like) available to the user computing devices.

In some implementations, users may provide a frequency at which data is collected and stored in the memory 256. For example, a care provider may provide a sensor assembly 202 configured to collect and store patient physiological data in the memory 256 every other second, every 10 seconds, every minute, every 5 minutes, every 10 minutes, every hour, every day, and the like. This can be advantageous in preventing the sensor assembly 202 from collecting and storing superfluous amount of data, especially in situations in which periodic measurement of, for example, blood oxygen level, blood pressure, heart rate, and the like, is sufficient. Additionally, by adjusting the frequency at which data is collected and stored in the memory 256, the life of the battery 224 and the memory 256 can be extended.

In some implementations, users may configure the sensor assembly 202 to specify a specific time period for collecting patient physiological data. For example, a care provider may wish to collect or measure blood glucose level between 7 a.m. and 10 a.m. every day. In another example, a care provider may wish to collect or measure blood oxygen level once during the morning between 8 a.m. and 10 a.m. and once during the evening between 6 p.m. and 8 p.m. Additionally, users may configure the sensor assembly 202 to specify data collection and storage frequency for a specific time period. The specific time period can be a specific day of a week, a day of a month, and the like. For example, a care provider can configure the sensor assembly 202 to measure heart rate every hour between 6 a.m. and 10 a.m., every 2 hours between 10 a.m. and 6 p.m., and every 3 hours between 6 p.m. and 6 a.m. As such, a sensor assembly 202 may be customized to collect patient physiological data depending on a patient's conditions and physiological data monitored. This can allow care providers to, for example, more easily identify general trends without having to search for data during specific time periods at specific intervals.

In some implementations, the memory 256 may store a default length of time for collecting and storing patient data and/or health-related events prior to establishing wireless communication with, for example, the computing system 206. As described herein, care providers may provide a configuration identifying a certain length of time for the sensor assembly 202 to collect and store patient data and/or health-related events. However, when no such configuration is provided, the sensor assembly 202 may access the default length of time from the memory 256 and proceed to collect and store patient data and/or health-related events. The default length of time may be configurable. The default length of time may vary between about 1 hour and about 30 days. In some implementations, the default length of time may be greater than 30 days.

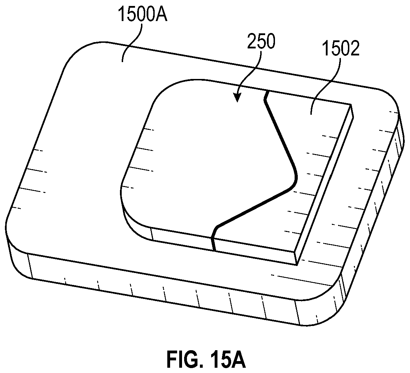

Since the battery 224 has a limited charge capacity, it may be depleted over time. Since the battery 224 provides power for the reusable module 250, depleted battery 224 may prevent the reusable module 250 from storing patient physiological data in the memory 256 and/or wirelessly transmitting data, for example, to the computing device 206 and/or a remote server. In some implementations, a backup power device 1500A may be used to provide power for the reusable module 250. An example of a backup power device 1500A is shown in Figure 15A.

The backup power device 1500A may be coupled to the reusable module 250. When coupled to the reusable module 250, the backup power device 1500, as described herein, can provide power for the reusable module 250. The reusable module 250 then can use the power from the backup power device 1500A to access patient physiological data stored in the memory 256 and wirelessly transmit the data to, for example, the computing device 206. The backup power device 1500 can be useful when a replacement disposable device is not readily available. The backup power device 1500A may include one or more electrical contacts that can come into contact with the electrical contacts 258 of the reusable module 250 and allow power to be transmitted from the backup power source 1500A to the reusable module 250. In some implementations, the backup power device 1500A can include a holder 1502 that can hold the reusable module 250 in place. Optionally, the holder 1502 can magnetically hold the reusable module 250 in place. Optionally, the backup power device 1500A may be mounted to a wall.

In some implementations, the backup power device 1500A has an internal power supply device and power from the internal power supply device can be used to provide power for the reusable module 250. Alternatively, as described herein, the backup power device 1500A may be mounted to a wall and can receive power from an external power source, for example, power grid of a building. The power from the external power source can be used to provide power for the reusable module 250.

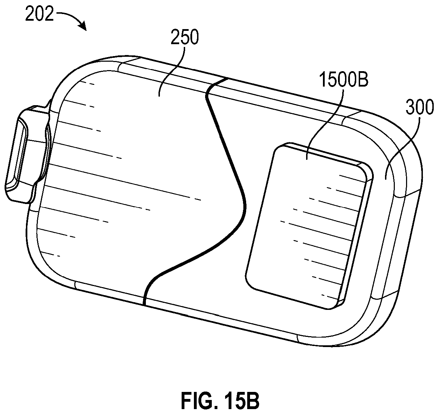

Additionally or alternatively, the backup power device 1500A may wirelessly provide power for the reusable module 250. As such, the backup power device 1500A may be a device with wireless charging capacity. In some implementations, as shown in Figure 15B, the backup power device 1500B may be magnetically coupled to the sensor assembly 202. In some implementations, the backup power device 1500B may be magnetically coupled to the reusable module 250 or to the disposable module 220 as shown in Figure 15B. The backup power device 1500 may be able to magnetically attach to the reusable module 250 while the reusable module 250 is coupled to the disposable module 220. As described herein, the backup power device 1500 may wirelessly provide power to the reusable module 250, which can in turn provide the power provided by the backup power device 1500 to the disposable module 220. As such, the backup power device 1500 may be used to power the sensor assembly 202 and allow the sensor assembly 202 to collect and wirelessly transmit patient physiological data. Alternatively, as described herein, the backup power device 1500 can attach to the disposable module 220, for example, the housing 300, to provide power for the batteries 224. As such, the backup power device 1500 may directly provide power for the disposable module 220, which can transmit power to the reusable module 250 for, for example, processing patient physiological data and/or wirelessly transmitting the data to, for example, the computing device 206.

According to the present invention the patient data and/or health-related events stored in the memory 256 vary in fidelity (that is, the degree to which the patient data collected by the sensor assembly 202 accurately reflects the actual patient data). In some implementations, the fidelity of the patient data (for example, heart rate) may vary based at least in part on the length of time the sensor assembly 202 is configured to collect and store patient data and/or health-related events. For example, the longer the length of time the sensor assembly 202 is configured to collect and store patient data and/or health-related events, the lower the fidelity of the patient data and/or health-related events may be, and vice versa. Such variance in fidelity may be caused by the limited storage capacity of the memory 256.

In some implementations, the fidelity may vary between different patient data or health-related events. In certain situations, certain patient data (for example, blood oxygen level) may be more important than other patient data (for example, body temperature). For example, for patients suffering from Malaria, it may be more important to monitor blood oxygen level more closely than other patient data such as heart rate or core body temperature. Accordingly, care providers may provide fidelity settings for the sensor assembly 202 that provides different level of fidelity for collecting patient data and/or health-related events. In some aspects, the data stored in the memory 256 can be transmitted to an outside server. The memory 256 can transfer the entire patient physiological information to the outside server or transmit only certain portions of the information. For example, the memory 256 can transmit timestamp information and associated event information to the external server. In another example, the memory 256 can transmit a snapshot of patient physiological information.

The processor 254 can be a chip, an expansion card/board, or a stand-alone device that interfaces with peripheral devices. For example, the processor 254 is a single integrated circuit on a circuit board for the reusable module 250. The processor 254 can be a hardware device or a software program that manages or directs the flow of data.

The processor 254 can communicate with the antenna 252 and the memory 256 of the reusable module 250. For example, the processor 254 communicates with the antenna 252 and the memory 256 of the reusable module 250 to retrieve or receive patient physiological data and to transmit the data to external devices via the antenna 252. The processor 254 can be a Bluetooth^{®} chipset. For example, the processor 254 is a SimpleLinkTM Bluetooth^{®} low energy wireless MCU (microcontroller unit) by Texas Instruments Incorporated.

The processor 254 of the reusable module 250 can be connected to the sensor 240 such that it receives patient physiological data from the sensor 240 when the reusable module 250 is mated with the dock 222. The processor 254 can retrieve the patient physiological data from the memory 226 of the dock 222 and transmit the data to the antenna 252. The processor 254 can be operatively connected to the antenna 252 such that the processor 254 can use the antenna 252 to wirelessly transmit the patient physiological parameters to the computing device 206. The patient physiological data transmitted from the reusable module 250 to the computing device 206 can be raw patient physiological data in analog format (for example, 1131001310113100) or patient physiological parameters in a digital format (for example, 60% SpO2).

The sensor 240 can transmit raw or analog patient physiological data to the processor 254 of the reusable module 250. The processor 254 can then perform signal processing on the raw data to calculate patient physiological parameters. It can be advantageous to have the processor 254 to perform signal processing on the raw patient physiological data instead of having the computing device 206 perform signal processing on the raw data. Raw data can comprise strings of binary bits, whereas processed data can comprise digital (not binary) data (for example, 36 degrees Celsius, 72 beats per minute, or 96% blood oxygen level). Therefore transmitting digital data can require less power consumption than transmitting raw data. Thus, by performing signal processing on the raw data using the processor 254 and transmitting the processed data (as opposed to raw data) to the computing device 206, life of the battery 224 can be extended.

The battery 224 of the dock 222 can provide power for the sensor 240. Additionally, the battery 224 can provide power for the reusable module 250. In some aspects, the reusable module 250 may not have an internal power source to transmit patient data to the computing device 206. When the reusable module 250 is mated with the dock 222, the processor 254 of the reusable module 250 can draw power from the battery 224. The processor 254 can use the power from the battery 224 to process patient physiological data from the sensor 240 and to wirelessly transmit the data to the computing device 206. The battery 224 may or may not be rechargeable. The battery 224 can have wireless charging capacity.

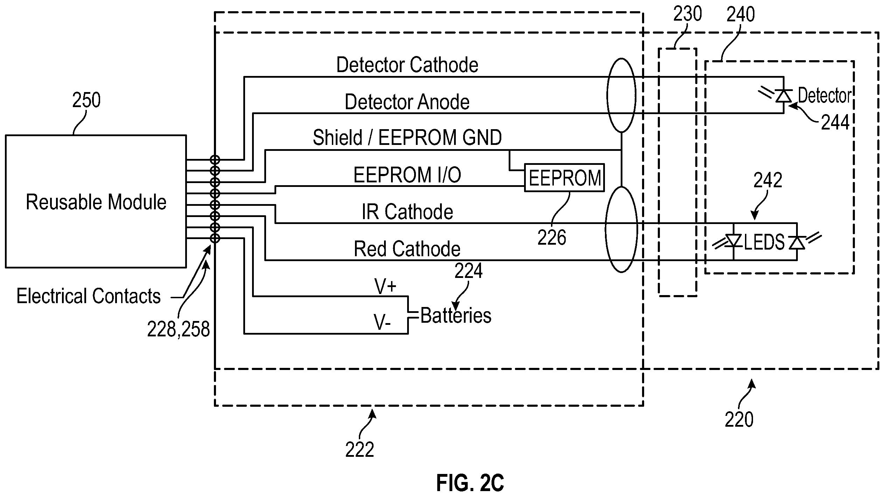

FIG. 2C illustrates a wiring diagram for the sensor system 202. The sensor 240 can include one or more detectors 244 and one or more emitters 242. The detectors 244 and the emitters 242 can be optical. The emitters 242 can be LEDs. The detectors 244 can detect light generated by the emitters 242. The emitters 242 and the detectors 244 are used to collect different types of patient physiological data, such as blood oxygen level, heart rate, and respiratory rate. As discussed below, the sensor 240 can include one of the following sensor elements including, but not limited to, piezoelectric elements for acoustic sensors, electrodes for EEG sensors, electrodes for ECG sensors, and the like.

The dock 222 and the reusable module 250 can include one or more electrical contacts 228 and electrical contacts 258, respectively. The electrical contacts 228 and 258 can establish electronic communication between the dock 222 and the reusable module 250 when the reusable module 250 is mated with the dock 222. The electrical communication between the electrical contacts 228 and 258 can allow the reusable module 250 to receive power from the battery 224 of the disposable module 220. Additionally and/or alternatively, the electrical connection between the electrical contacts 228 and 258 can allow the reusable module 250 to receive patient physiological data from the memory 226 of the dock 222. In some implementations, the reusable module 250 receives the patient physiological data from the sensor 240 such that the memory 226 does not store the patient physiological data. The coupling of the reusable module 250 and the dock 222 will be further described below.

### Sensor Assembly

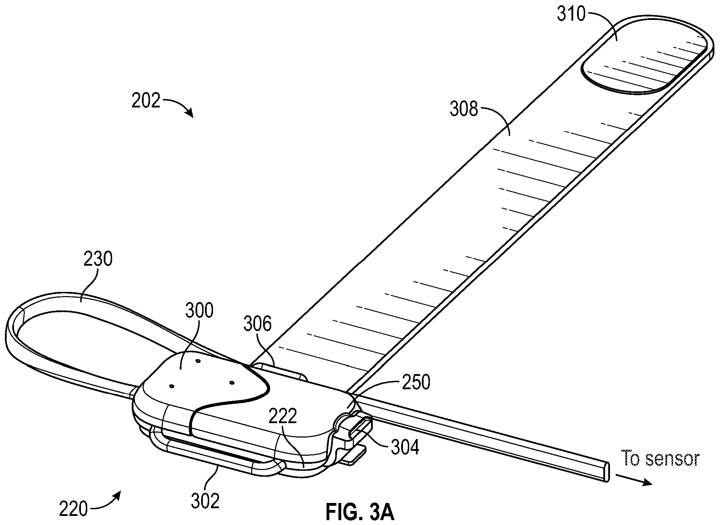

FIG. 3A shows a front perspective view of an example of the sensor assembly 202 including the reusable module 250 and the disposable module 220. As discussed above, the reusable module 250 can be a pairing device that can establish wireless connection with the computing device 206. The disposable device 220 can include the dock 222 and the cable 230 coupling the dock 222 to the sensor 240 (not shown).

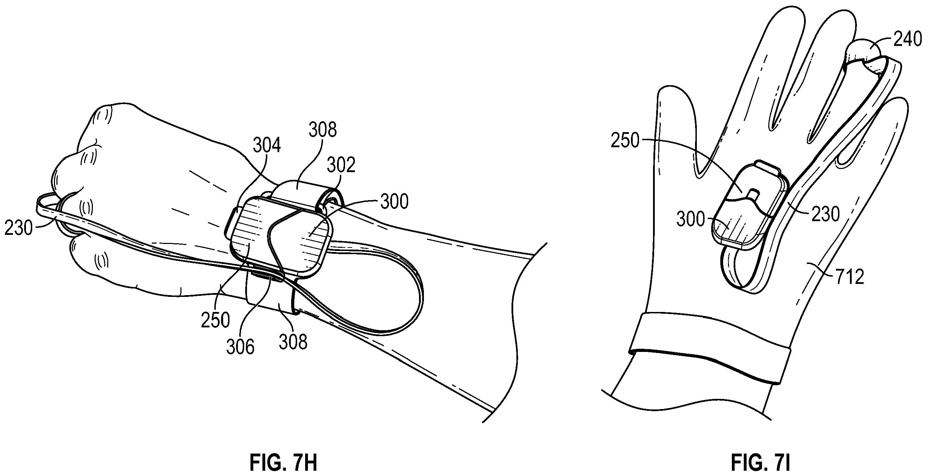

The dock 222 can include a strap 308 that is coupled to a bottom portion of the dock 222. The strap 308 can loop around a patient (e.g., a wrist or an arm) to removably attach the dock 222 to the patient (see FIG. 7H). The dock 222 can also include a strap loop 302 having a slot for the strap 308 to extend through. The strap 308 can extend through the strap loop 302 and loop around to removably attach the dock 222 to the patient. The strap 308 can include a fastener 310 disposed near a distal end of the strap 308 that can interact with the strap 308 to fix the distal end of the strap 308. The fastener 310 can be located at a distal end of the strap 308, as shown in FIG. 3A. The fastener 310 can be located at other locations of the strap 308. The dock can also include a retainer 304 that holds the reusable module 250 within the dock 222 to maintain electrical connection between the reusable module 250 and the dock 222. Moreover, the dock 222 can include a housing 300 that can house the battery 224 and the memory 226.

The dock 222 can include a cable retainer 306 disposed on a side of the dock 222. The cable retainer 306 can be dimensioned and sized to retain the cable 230. The cable retainer 306 can be removably connected to the dock 222. At least a portion of the cable retainer 306 may be flexible to facilitate insertion of the cable 230 into the cable retainer 306. The cable retainer 306 can advantageously limit movement of the cable 230 to prevent possible tangling of cables of different sensor assemblies. The cable retainer 306 can include a channel to through which the cable 230 can extend. The channel of the cable retainer 306 can be dimensioned such that the cable 230 is snug within the channel, thereby limiting movement of the cable 230.

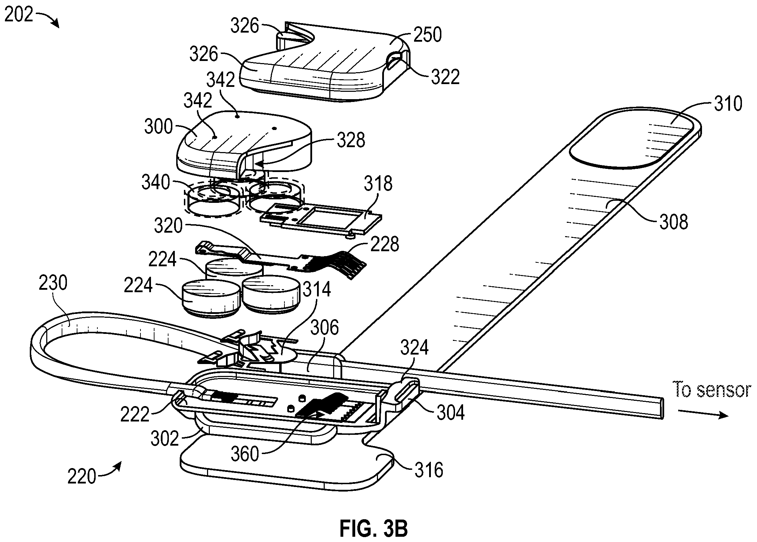

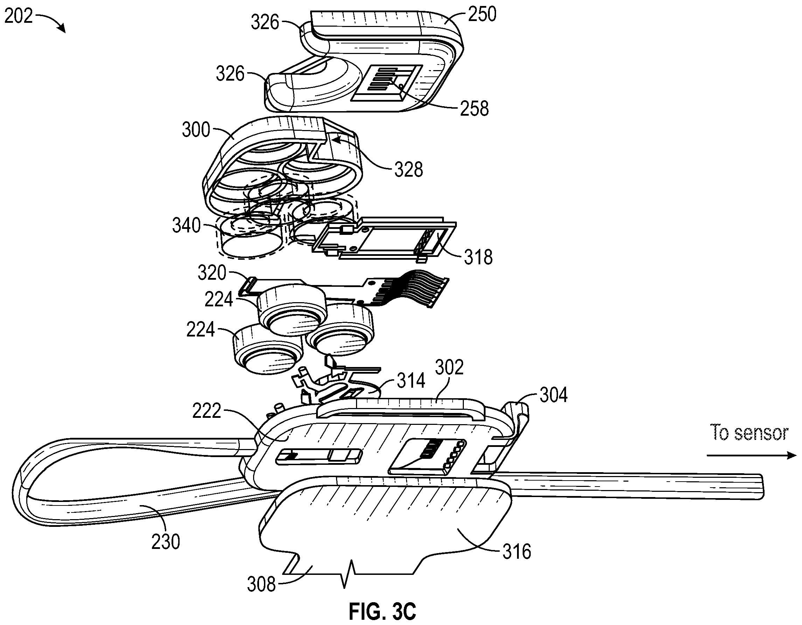

FIG. 3B illustrates an exploded, top perspective view of the sensor assembly 202 of FIG. 3A. FIG. 3C illustrates an exploded, bottom perspective view of the sensor assembly 202 of FIG. 3A. The dock 222 of the disposable module 220 can include a support plate 316 disposed under the dock 222. The support plate 316 can be integrated with the strap 308. The strap 308 can be modular with respect to the support plate 316 and/or the dock 222. The dock 222 may not include the support plate 316 such that the strap 308 is coupled directly to the dock 222.

The retainer 304 of the dock 222 can include a protrusion 324 that can interact with a groove 322 of the reusable module 250. The interaction between the groove 322 and the protrusion 324 can maintaining coupling between the reusable module 250 and the dock 222. For example, when the reusable module 250 is inserted into the dock 222, the retainer 304 is pushed in a direction away from the housing 300 of the dock 222 in order to allow the reusable module 250 to mate with the dock 222. When the reusable module 250 is fully inserted into the dock 222, the retainer 304 can snap back to its original position to engage the groove 322 of the reusable module 250. The retainer 304 and the groove 322 can together prevent vertical displacement of the reusable module 250.

The retainer 304 can have a first position and a second position. When in the first position, the retainer 304 is substantially vertical with respect to the dock 222. When in the second position, the retainer 304 is pushed in a direction away from the housing 300 so that the retainer 304 forms an angle greater than 90 degrees with respect to the dock 222. Before the reusable module 250 is inserted into the dock 222, the retainer 304 can be in the first position. While the reusable module 250 is being pushed into the dock 220, the reusable module 250 interacts with the retainer 304 and causes the retainer 304 to be in the second position. When the reusable module 250 is fully engaged with the dock 222, the retainer 304 reverts to the first position so that the protrusion 324 engages the groove 322.

The dock 222 can also include a flex circuit 320 and a cover 318 to retain the flex circuit 320. The flex circuit 320 can include the electrical contacts 228 of the dock 222, where the flex circuit 320 serves as a connection between the cable 230 and the electrical contact 228. Therefore any information or data transmitted from the sensor 240 via the cable 230 to the dock 222 can be transmitted to the electrical contacts 228 via the flex circuit 320. Additional details of the flex circuit 320 will be provided below.

The housing 300 of the dock 222 can include one or more slots 328 that can interact with one or more legs 326 of the reusable module 250. The slots 328 can be dimensioned and shaped to allow the legs 326 of the reusable module 250 to slide into the slots 328. The legs 326 can slide into the slots 328 to assist in maintaining connection between the reusable module 250 and the dock 222. Once the legs 326 are inserted into the slots 328, the legs 326 can prevent vertical displacement of the reusable module 250.

It can be advantageous to have the battery 224 in a disposable portion such as the dock 222 or the sensor 240. Establishing wireless communication 204 and performing wireless transmission requires a significant amount of power. If the reusable module 250 has an internal power source, its functionalities (for example, establishing wireless communication 204 and performing wireless transmission) can be limited by the capacity of the internal power source. In such configuration, the reusable module 250 needs to be replaced once its internal power source is depleted. In a wireless patient monitoring context, it is desirable to keep the same pairing device for each patient because having to use multiple pairing devices for the same patient often can lead to confusion and can create a need to reestablish connections between pairing devices and display devices. When the reusable module 250 has an external power source such as battery 224 of the dock 222, it does not need to be replaced when the battery 224 is depleted.

The batteries 224 can be zinc-air batteries powered by oxidizing zinc with oxygen in the air. It can be advantageous to use zinc-air batteries because they have higher energy density and thus have greater capacity than other types of batteries for a given weight or volume. In addition, zinc-air batteries have a long shelf life if properly sealed to keep the air out. The housing 300 can include one or more openings 332 that allow air to enter and react with the batteries 224. The one or more openings 332 can be sealed prior to use to prevent the air from entering and reacting with the batteries 224, thereby reducing capacity of the batteries 224. Once ready to use, the seal placed on the one or more openings 332 may be removed to allow the batteries 224 to provide power for the reusable module 250. The housing 300 may include a gasket 330 to seal the batteries 224 from the air. The gasket 330 can further increase the capacity of the batteries 224.

Having a disposable element (for example, the disposable module 220) as a power source for the reusable module 250 can address the above issues by eliminating the need to replace the reusable module 250. In this configuration, only the dock 222 or the sensor 240 needs to be replaced when the battery 224 is depleted. Since the cost of replacing the dock 222 or the sensor 240 can be much less than the cost of replacing the reusable module 250, this configuration can be advantageous in reducing operation costs. The sensor 240 may include the battery 224 that provides power to the reusable module 250. Both the sensor 240 and the dock 222 can include the battery 224. The reusable module 250 can include a battery consumption priority setting such that the reusable module 250 receives power first from the sensor 240 then from the dock 222.

The dock 222 can include a battery circuit 314 in contact with the batteries 224. The battery circuit 314 can be in contact with the flexible circuit 320. When the reusable module 250 is mated with the dock 222, the electronic contacts 258 can be in contact with the electronic contacts 228 of the flexible circuit 320 to allow the reusable module 250 to receive power from the batteries 224 via the flexible circuit 320.

The dock 222 can include an opening 362 and one or more supports 360. The one or more supports 360 can be formed on a side of the opening 362 and extend over a substantial portion of the opening 362. The supports 360 can be arcuate. The supports 360 can extend over the length of the opening 362. The cover 318 for the flexible circuit 320 can be placed over the opening 362 to hold the flexible circuit 320 over the opening 362.

The dock 222 can include a slot dimensioned to retain the reusable module 250 during the use of the sensor assembly 202. The reusable module 250 can be disposed between the housing 300 and the retainer 304. The slot of the dock 222 can include one or more arcuate surfaces or one or more angular corners. The slot of the dock 222 may be substantially rectangular or circular in shape. The slot can have substantially the same size , shape, and/or dimensions as that of the reusable module 250.

The reusable module 250 can include one or more electrical contacts 258. The electrical contacts 258 can be located on a bottom surface of the reusable module 250. The electrical contacts 258 can be substantially rectangular or circular in shape. The electrical contacts 258 can establish contact with electrical contacts 228 of the dock 222 when the reusable module 250 is mated with the dock 222. The contact between the electrical contacts 228 and electrical contacts 258 can allow information or data be transmitted between the reusable module 250 and the dock 222 of the disposable module 220.

As disclosed herein, the batteries 224 can be zinc-air batteries powered by oxidizing zinc with oxygen in the air. The openings 332 formed on the housing 300 can allow the air to enter through and react with the battery 224. The battery 224 then provides power for the disposable module 220 and the reusable module 250. However, the openings 332 may sometimes be covered by blankets, clothes, and the like, which can prevent the air from entering through the openings 332 and react with the battery 224. Consequently, power supply for the disposable module 220 and the reusable module 250 can be interrupted if the openings 332 are covered.

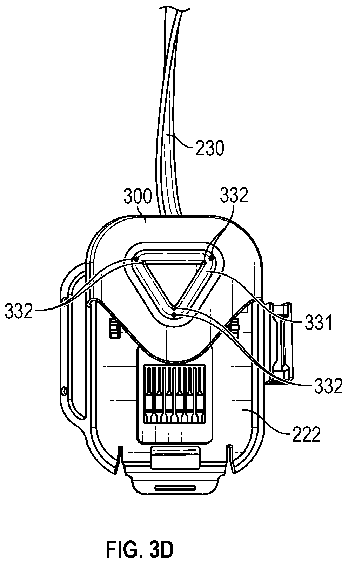

As shown in FIG. 3D, the housing 300 can include one or more recesses 331, such as, for example, channels, that can facilitate the air to enter through the openings 332. The recesses 331 can be formed on a top surface of the housing 300 such that the recesses 331 form openings that allow air flow. The openings 332 may be formed on an inner surface of the recesses 331. The inner surfaces of the recesses 331 are at least a predetermined distance away from the top surface of the housing 300 so that even when the housing 300 is covered, the openings 332 may remain uncovered and exposed to the air. The housing can have a single channel or multiple recesses, such as dimples or cutouts of any shape or size.

The number, dimensions, orientation, or positions of the channels 331 may be varied depending on the size of the housing 300 of the reusable module 250. The channels 331 can be oriented such that they together form a shape on the housing 300. The channels 331 may be oriented in a triangular shape (as shown in FIG. 3D), rectangular shape, pentagonal shape, hexagonal shape, and the like. The cross-sectional shape of the channels 331 can be circular, triangular, rectangular, or the like. In some examples, the channels 331 can extend to one or more edges of the housing 300 so that even when the top surface of the housing 300 is covered, the channels 331 extending to the edges of the housing 300 can ensure that the openings 332 remain exposed to the air.

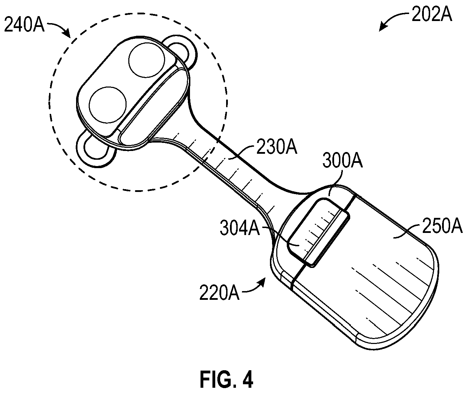

FIG. 4 illustrates an example the sensor assembly 202, identified generally by the reference numeral 202A. Parts, components, and features of the sensor assembly 202A are identified using the same reference numerals as the corresponding parts, components, and features of the sensor assembly 202, except that a letter "A" has been added thereto. The illustrated example includes a disposable module 220A and a reusable module 250A coupled to each other.

The sensor assembly 202A can include a sensor 240A. The sensor 240A can be an O3 sensor that can be adhered to a forehead of a patient. The sensor assembly 202A can include a cable 230A that couples the sensor 240A and a dock 222A of the disposable module 220A. The cable 230A can be flat or round. As discussed above, the sensor 240A can include one or more batteries that can provide power for a reusable module 250A. The mating of the dock 222A and the reusable module 250A can facilitate electronic communication therebetween. The dock 222A can include a housing 300A that includes a retainer member 304A. Pressing down the retainer member 304A can allow the reusable module 250A to be coupled with or removed from the dock 222A.

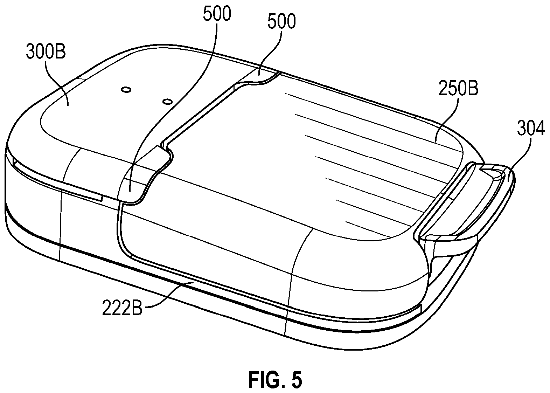

FIG. 5 illustrates an example of the sensor assembly 202, identified generally by the reference numeral 202B. Parts, components, and features of the sensor assembly 202B are identified using the same reference numerals as the corresponding parts, components, and features of the sensor assembly 202, except that a letter "B" has been added thereto. The illustrated example includes a disposable module 220B and a reusable module 250B coupled to each other.

The sensor assembly 202B can include a sensor 240B. The sensor 240B can be a RAM sensor adhered to a neck of a patient. The sensor 240B can be an ECG sensor that can be adhered to a chest or abdominal area of a patient. The dock 222B can include a housing 300B and a retainer member 304B. The housing 300B can include one or more extensions 500 that can extend from the body of the housing 300B towards the retainer member 304B. The reusable module 250B can include cutouts that correspond to the one or more extensions 500. When the reusable module 250B is coupled with the dock 222B, the extensions 500 can extend over the cutouts of the reusable module 250B, preventing the reusable module 250B from being dislodged from the dock 222B.

### Flexible Circuit

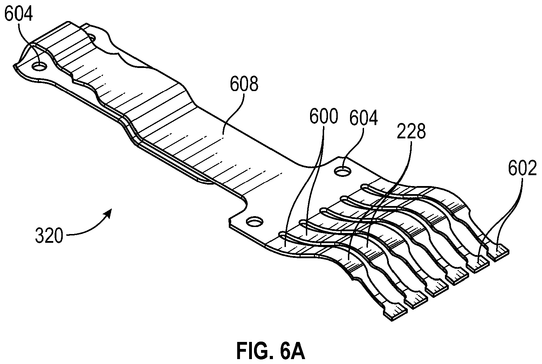

FIG. 6A illustrates a perspective view of the flex circuit 320. The flex circuit 320 can include one or more elongate members 600 that can each include a tip 602, and a body 608. The electrical contracts 228 can be disposed on the one or more elongate members 600. The elongate members 600 can extend distally from the body 608. The tips 602 can be located at distal ends of the elongate members 600 of the flex circuit 320. The elongate members 600 can be flat or arcuate as shown in FIG. 6A. The elongate members 600 can become arcuate due to their interaction with the supports 360 and the cover 318. The elongate members 600 can include one or more substantially flat portions and/or one or more arcuate portions. Each of the one or more tips 602 can correspond to each of the one or more elongate members 600 of the flex circuit 320. Some of the elongate members 600 may not have electrical contacts 228. The flex circuit 320 can include the same or different number of the elongate members 600 and the tips 602. The flex circuit 320 can include one or more openings 604 that couple the flex circuit 320 to the dock 222.

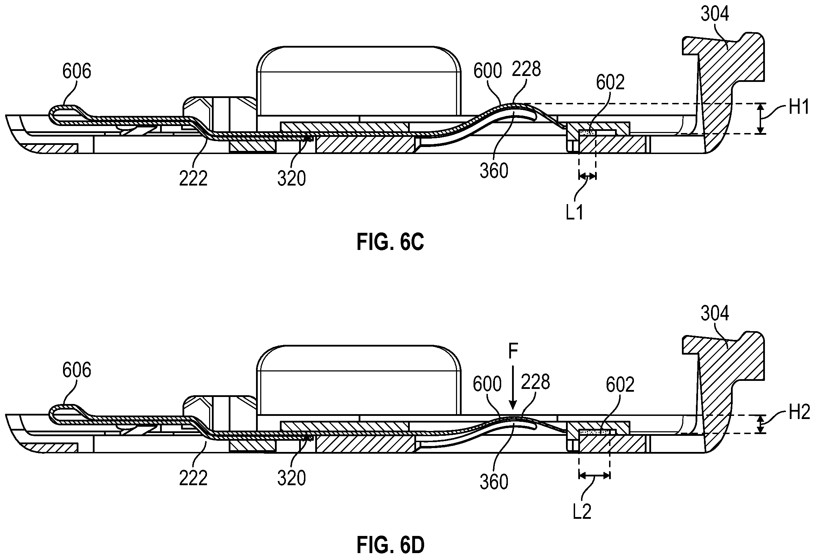

As shown in FIGs. 6C and 6D, the tips 602 of the elongate members 600 can be positioned under the cover 318 while the elongate members 600 are supported by supports 360. Because the tips 602 can be wedged under the cover 318, the elongate members 600 can retain its arcuate shape over the supports 360.

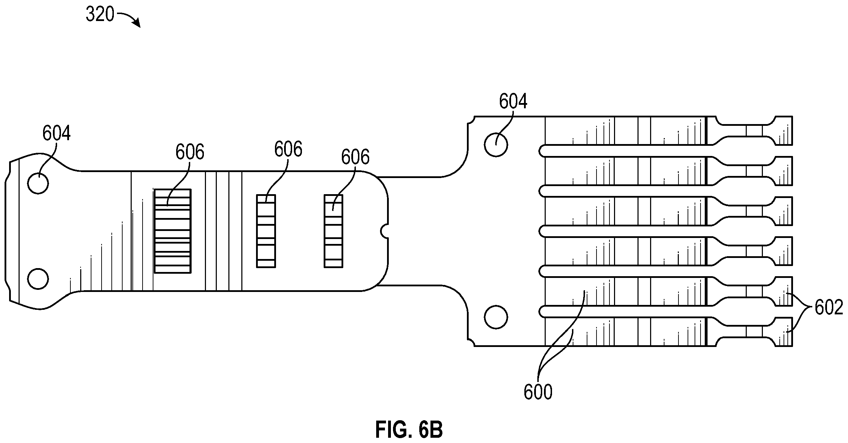

FIG. 6B illustrates a bottom view of the flex circuit 320. The flex circuit 320 can include one or more electrical contacts 606 that can be connected to the cable 230 and the battery circuit 314 (see FIGs. 3A and 3C). Therefore, power from the battery 224 can be transmitted to the electrical contacts 228 of the dock 222 via the electrical contacts 606 of the flex circuit 320. Moreover, the electrical contacts 606 can establish connection between the electrical contacts 228 and the sensor 240 via the cable 230.

The number of the elongate members 600 can correspond to the number of electrical contacts 258 of the reusable module 250 (see FIG. 3C). For example, the reusable module 250 has six electrical contacts 258 and the flex circuit 320 has six fingers, where each of the six fingers includes an electrical contact 228. The number of electrical contacts 258 of the reusable module 250 can be different from the number of elongate members 600 of the flex circuit 320. For example, the flex circuit 320 can include six elongate members 600 each having a corresponding electrical contact 310a, while the reusable module 250 has only four electrical contacts 258. The number of electrical contacts 258 of the reusable module 250 may be different from or the same with the number of electrical contacts 228 disposed on the elongate members 600 of the flex circuit 320.

Each the elongate members 600 of the flex circuit 320 can include an arcuate portion with a first curvature. The arcuate portions of the elongate members 600 can be laid over the opening 362 of the dock 222. The one or more electrical contacts 228 of the flex circuit 320 can be disposed over a portion of the elongate members 600 of the flex circuit 320. For example, the one or more electrical contacts 228 are located at an apex of each of the elongate members 600 of the flex circuit 320. In another example, the entire upper surface of each of the elongate members 600 defines the electrical contacts 228. The elongate members 600 of the flex circuit 320 can be configured such that the apex of the arcuate portions of the elongate members 600 of the flex circuit 320 are located at a predetermined distance away from the opening 362 of the dock 222. The apex of the elongate members 600 of the flex circuit 320 can point away from the opening 362 of the dock 222 such that the arcuate portions of the elongate members 600 define a concave surface facing the opening of the dock 222. The apex of the elongate members 600 can be arcuate in shape or substantially flat.

It can be advantageous to have the elongate members 600 of the flex circuit 320 include a curved portion upward and away (for example, concave downward) from the opening 362 of the dock 222. Such configuration can allow the elongate members 600 to act as springs providing reactive upward forces when pressed downward by the reusable module 250. Such upward forces provided by the elongate members 600 can allow the electrical contacts 228, 258 of the dock 222 and the reusable module 250, respectively, to maintain adequate contact between them.

The elongate members 600 of the flex circuit 320 can have different curvatures. For example, a first elongate member of the flex circuit 320 has a first curvature while a second elongate member of the flex circuit 320 has a second curvature. The first curvature of the first elongate member and the second curvature of the second elongate member can be the same or different. The first curvature of the first elongate member is greater than, less than, or equal to the second curvature of the second elongate member.

The elongate members 600 of the flex circuit 320, in their resting positions, may not have any arcuate portions. The elongate members 600 of the flex circuit 320 can be substantially linear prior to being installed on the dock 222. The elongate members 600, can be linear or curved. The elongate members 600 of the flex circuit 320 can include more than one linear portions.

The elongate members 600 of the flex circuit 320 can be flexible or not flexible. The flex circuit 320 can be laid on the dock 222 such that the elongate members 600 are laid over one or more supports 360 of the dock 222. The elongate members 600 can extend distally away from the body 608 of the flex circuit 320. The flex circuit 320 can include more than one elongate members 600. The flex circuit 320 can include one or more elongate members 600 that are flexible. Some the elongate members 600 may be flexible while other elongate members 600 are not.

As discussed above, the dock 222 can include the opening 362 over which the elongate members 600 of the flex circuit 320 can extend over. The dock 222 can include one or more supports 360 dimensioned and shaped to support the elongate members 600 of the flex circuit 320. When the flex circuit 320 is installed on the dock 222, the supports 360 can provide a surface on which the elongate members 600 of the flex circuit 320 can be placed on.

The supports 360 of the dock 222 can be curved and define the curvature of the arcuate portions of the elongate members 600. The supports 360 can be arcuate. It can be advantageous to have the supports that correspond to each of the elongate members 600 of the flex circuit 320. For example, the dock 222 has six independent supports 360 associated with each of the six elongate members 600 of the flex circuit 320. Such configuration allows each of the corresponding elongate members 600 and the supports 360 of the dock 222 to move independently from other elongate members 600 and supports 360 as opposed to all of the elongate members 600 and the supports 360 moving that the same time. Such configuration can make inserting the reusable module 250 into the slot 940 of the dock 222 easier. Moreover, this can allow interoperability between the dock 222 and the reusable module 250 that have different height configurations for the electrical contacts 258.

It can be advantageous to have the supports 360 for the flex circuit 320 include a curved portion upward and away (e.g., concave downward) from a bottom portion of the dock 222. Such configuration can allow the supports to act as springs providing reactive upward force when pressed downward by the reusable module 250. Such upward forces can allow the electrical contacts 228, 258 of the dock 222 and the reusable module 250, respectively, to maintain adequate contact between them. The supports 360 can include a first upward portion that is concave upward, a second upward portion that is concave downward, and a third downward portion that is concave downward. The supports 360 may include a first upward portion that is concave upward and a second upward portion that is concave downward. The supports 360 can include one or more inflection point, defined as a point where the supports 360 changes from being concave to convex, or vice versa. The supports 360 can also include one or more linear portions.

The supports 360 may also provide sufficient force to push the reusable module 250 away the dock 222 when the retainer member 304 is pulled away from the reusable module 250. The support 360 may push the reusable module 250 away from the dock 222 when the retainer member 304 is in its second position, as discussed above. When the retainer 304 no longer engages the groove 322 of the reusable module 250, it may no longer provide force to counteract the force generated by the supports 360, allowing the supports 360 to push the reusable module 250 away from the dock 222.

The supports 360 can have a length that is greater than, less than, or equal to the length of the elongate members 600 of the flex circuit 320. The supports 360 have a width that is greater than, less than, or equal to the width of the elongate members 600. The supports 360 can have a thickness that is greater than, less than, or equal to the thickness of the elongate members 600 to allow the supports 360 to provide sufficient mechanical support and to withstand the downward force exerted on the elongate members 600 and the supports 360 by the reusable module 250. The interaction between the elongate members 600, supports 360, and the reusable module 250 will be further described below.

The supports 360 can be made out of the same or different material as the dock 222.

The body 608 of the flex circuit 320 can be laid under the housing 300 of the dock 222. The body 608 can be connected to the cable 230 connected to the dock 222 such that the flex circuit 320 allows the health monitoring data from sensor 240 to be transmitted to the electrical contacts 606 of the flex circuit 320.

FIGs. 6C and 6D illustrate a change in a configuration of the flex circuit 320. When the reusable module 250 is inserted into the slot 940 of the dock 222, the engagement between the reusable module 250 and the dock 222 can change the position of the tips 602 of the flex circuit 320. FIGs. 6C and 6D show relative positions of the tips 602 before and after the reusable module 250 is mated with the dock 222. The relative positions of the tips 602 before the reusable module 250 is inserted into the dock 222 are denoted by L1. When the reusable module 250 is inserted into the slot 940 of the dock 222, the reusable module 250 can apply a downward force (denoted as F) to the arcuate portions of the elongate members 600 and the supports 360. This downward force F can cause the arcuate portions and the supports 360 to move downward. This downward movement of the elongate members 600 and the supports 360 can cause the tips 602 to move distally along an axis defined by the elongate members 600 of the flex circuit 320. Specifically, such downward motion can cause the relative positions of the tips 602 to change from L1 to L2, where L2 is greater than L1.

FIGs. 6C and 6D illustrate another change in configuration of the flex circuit 320. When the reusable module 250 is inserted into the dock 222, the engagement between the reusable module 250 and the dock 222 can change the position of the tips 602 of the flex circuit 320. The relative difference between the heights of the apex of the arcuate portions of the elongate members 600 and the body 608 before for reusable module 250 is inserted is denoted by H1. When the reusable module 250 is inserted into the dock 222, the reusable module 250 can apply a downward force (denoted as F) to the arcuate portions of the elongate members 600 and the supports 360. This downward force F can cause the arcuate portions and the supports 360 to move downward. Such downward motion can cause the relative difference between the heights of the apex of the arcuate portions of the elongate members 600 and the body 608 to change from H1 to H2, where H2 is less than H1. It is possible that the relative different between the heights of the apex of the arcuate portions of the elongate members 600 and the body 608 can change while the relative positions of the tips 602 do not change from L1 to L2, or vice versa.

The downward force F in a first direction can cause the supports 360 of the dock 222 to provide a reactive force in a second direction. The second direction of the reactive force can be an opposite direction then the first direction of the downward force F. Specifically, the reactive force by the supports 360 can be upward away from the dock 222. The supports 360 can act as a spring such that as the supports 360 moves further downward from its natural position (for example, as H1 changes to H2), the magnitude of the reactive force increases. The directions of F and the reactive force may be opposite from each other. The magnitude of the reactive force is less than the downward force F in order to allow the supports 360 to move downward and allow the reusable module 250 to be inserted into the slot 940 of the dock 222. The magnitude of the downward force F caused by the reusable module 250 may correlate to the following: the change in the relative height difference between the apex of the elongate members 600 and the body 608 (for example, from H1 to H2) and the change in the positions of the tips 602 (for example, from L1 to L2).

The elongate members 600 of the flex circuit 320 can have a first degree of curvature before the reusable module 250 is inserted into the dock 222. The elongate members 600 can have a second degree of curvature after the reusable module is inserted into the dock 222. The first degree of curvature of the elongate members 600 can be greater than, less than, or equal to the second degree of curvature. The first degree of curvature can correspond to a first position of the tips 602 (for example, L1). The second degree of curvature can correspond to a second position of the tips 602 (for example, L2). Moreover, the first degree of curvature can correspond to a first position of the apex (for example, H1) of the elongate members 600. The second degree of curvature can correspond to a second position of the apex (for example, H2) of the elongate members 600.

The reactive force provided by the supports 360 can maintain sufficient contact between the electrical contacts 310a of the dock 222 and the electrical contacts 310b of the reusable module 250 to allow electrical signals be transmitted between the contacts.

### Attachment Mechanisms

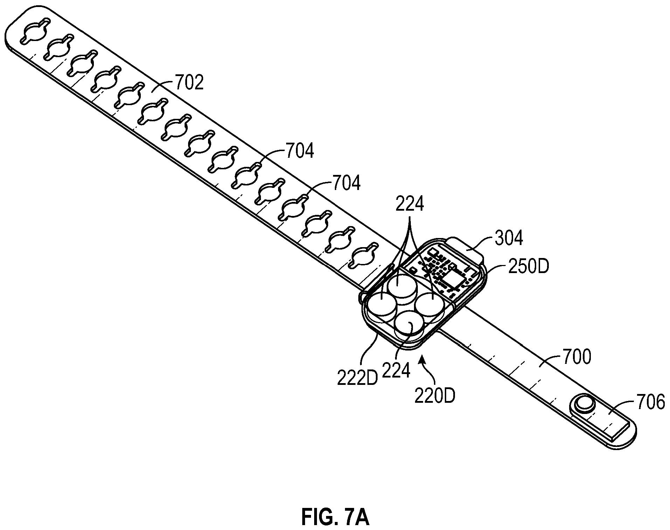

FIGs. 7A-7I illustrate various examples of an attachment mechanism for the disposable module 220 of the sensor assembly 202.

With reference to FIGs. 7A-7C, the dock 222 can be coupled to a first strap 700 and a second strap 702. The first strap 700 and the second strap 702 can be mechanically coupled to the dock 222. The straps 700, 702 may be removably coupled to the dock 222. Alternatively, the straps 700, 702 can be integrated to the dock 222. The second strap 702 can include one or more openings 704. The first strap 700 can include a fastener 706 configured to affix the second strap 702 to the first strap 700. The openings 704 can be dimensioned receive the fastener 706. The first strap 700 can be inserted through one of the openings 704 to removably attach the dock 222 to a patient. The straps 700, 702 can have varying thicknesses, lengths, and flexibility. The straps 700, 702 may be stretchable. The first strap 700 can include one or more openings 704 while the second strap 702 includes the fastener 706.

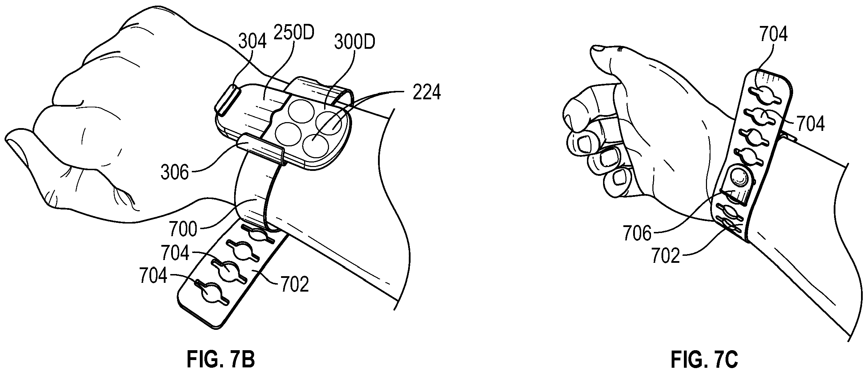

A distal end of the first strap 700 can be inserted into one of the openings 704 of the second strap 702. The fastener 706 of the first strap 700 may be inserted into one of the openings 704 of the second strap 702. The interaction between the fastener 706 and openings 704 can removably affix the dock 222 as shown in FIGs. 7B and 7C.

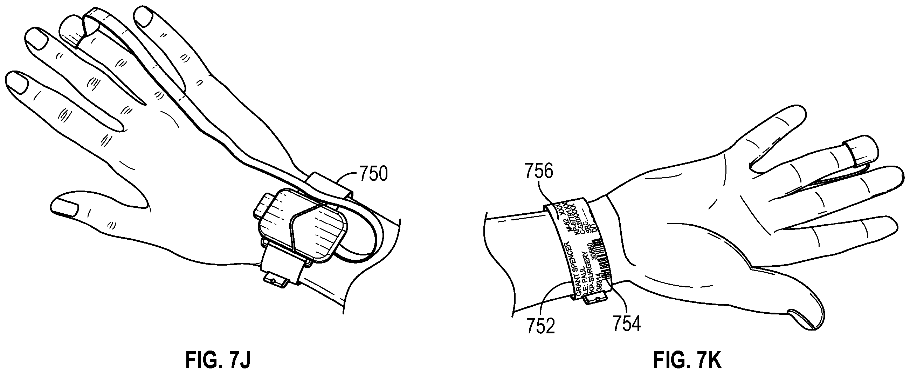

In some implementations, the sensor assembly 202 can be coupled to a hospital band 750 as shown in Figures 7J and 7K. The band 750 may include any of straps disclosed herein or any suitable strap or band to attach the sensor assembly 202 to a patient. The sensor assembly 202 may not include the strap (e.g., the strap 308 shown in FIG. 3B) and the dock 222 of the disposable module 220 may be coupled to the band 750. In some implementations, the dock 222 may include two strap loops (e.g., the strap loop 302) and the band 750 may be fed through the strap loops to couple the sensor assembly 202 to the band 750. The band securement mechanism can be non-removable once attached to the patient, requiring the band to be cut in order to be removed. The band can also include tamper detections and alarms which indicate the band has been removed improperly or tampered with. The band may include patient identifying information 752, a bar code 754, and medication information 756. The patient identifying information 752 can include the name of a patient, a contact information, doctor information, and the like. The bar code 754 can represent the patient identifying information 752 and may serve as an identifier that can be used to associate a patient with one or more devices (for example, patient monitoring devices). The bar code 754, in some example, may be a QR code. The medication information 756 may identify, for example, allergy information of a patient, medications provided to the patient, dosage information, and the like. In some implementations, the band 750 can include an RFID (radio frequency identification) tag that can, for example, allow patients and/or family members to get through security checkpoints. Optionally, the sensor assembly 202 may be wrist-mounted and include a one or more physiological sensors measuring parameters at the wrist. Optionally, the band 750 can include a location determination device that can determine exact or approximate locations of a patient.

By coupling the sensor assembly 202 to, for example, a hospital band 750, a number of items worn by a patient can be reduced. For example, if a patient is already wearing a medical wearable device (e.g., wearable fitness trackers, smart health watches, wearable ECG monitors, wearable blood pressure monitors, and the like), the sensor assembly 202 may be coupled to (e.g., adhered via adhesives or coupled via strap loops) the medical wearable device. Additionally or alternatively, the sensor assembly 202 may be coupled to non-medical devices such as a traditional watch or jewelry that may be worn on the wrist or other parts (e.g., ankle, neck, arm, leg, and the like) of a patient's body.

In some implementations, at least one of the patient identifying information 752, the bar code 754, or the medication information 756 may be printed separately and attached to, for example, the strap 308 shown in FIG. 3B of the sensor assembly 202. This configuration can obviate the need to, for example, detach the strap 308 from the dock 222 of the sensor assembly 202 and attaching the band 750 to the dock 222 (e.g., looping the band 750 to the strap loops 302 of the dock 222).

In some implementations, the appearance of the band 750 can include one or more light sources (e.g., light emitting diodes) that may be actuated (e.g., switched on/off)to reflect, for example, changes in patient condition. Optionally, various colors can be used to denote different patient conditions. For example, the color green may represent good and/or excellent patient condition, whereas the color yellow and the color red may represent suboptimal and critical patient conditions, respectively. The band 750 can include a processor and a wireless communication module that can receive physiological data of the patient wearing the band 750 from the sensor assembly 202 and cause the light sources to change the appearance of the band 750 based at least in part on the physiological data. In some implementations, the processor of the band 750 may receive patient status data (e.g., great, good, suboptimal, bad, critical, and the like) from the sensor assembly 202 (as opposed to receiving raw or processed patient physiological data) and change the display of the light sources based on the patient status data. This can be advantageous when a patient and/or care providers (or family members) do not have access to the patient's physiological data. By monitoring the appearance (for example, the color) of the band 750, patients, care providers, and family members can easily recognize and monitor patient conditions.

Optionally, the identifier can be used as a security tag that can cause an alarm system to generate an alarm if the identifier passes a geo-fence location. For example, a hospital or a care provider facility may have, for example, RF scanners located at various locations. Such RF scanners may be installed at a main entrance of a care provider facility or at certain checkpoints for, for example, emergency rooms, intensive care units, maternity wards, neonatal units and the like. RF scanner may detect the identifier of the hospital band attached to the sensor assembly 202 and generate an alarm. Such use of the identifier as security tags can prevent unauthorized and/or accidental movement of patients, removal or movement of the hospital bands with the sensor assembly 202, leaving the hospital without returning the sensor device and the like.

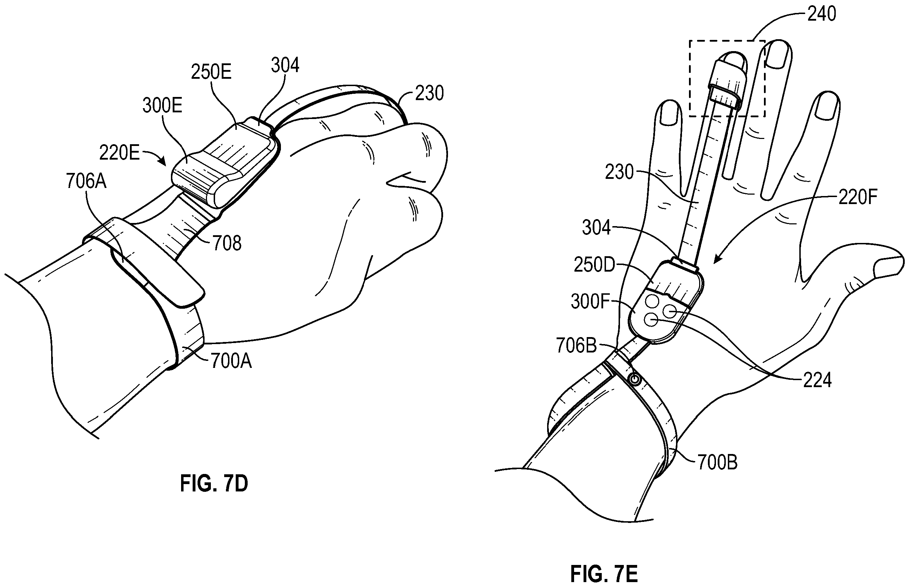

FIG. 7D shows the dock 222 of the disposable module 220 coupled to yet another example of an attachment mechanism. The dock 222 can be coupled to an extension 708 extending away from the disposable module 220. For example, as shown in FIG. 7D, the disposable module 220 can be placed on top of a hand and the extension 708 can extend towards a wrist of a patient. The extender 708 can include a strap 700A that can loop around the wrist to secure the disposable module 220 and the extension 708 to the wrist. The strap 700A can include a fastener 706A that can adhere the strap 700A to a top surface of the extension 708. The fastener 706A can be disposed at a distal end or a proximal end of the strap 700A. The fastener 706A may adhere to a top surface or a bottom surface of the 700A. The fastener 706A can incorporate one of the following mechanisms including a hook and loop system, Velcro, buttons, snaps, magnets, and the like.

FIG. 7E illustrates another example of an attachment mechanism for the disposable module 220. As shown here, the dock 222 can be coupled to a strap 700B. A first, proximal end of the strap 700B can be attached to the dock 222, while a second, distal end of the strap 700B can extend away from the dock 222. The distal end of the strap 700B can include a fastener 706B. The strap 700B can affix the dock 222 to a wrist of a patient by having the second, distal end looped around the wrist. The distal end of the strap 700B can be affixed by looping over or under the proximal end of the strap 700B. Once the distal end of the strap 700B looped around the first, proximal end of the strap 2310, the fastener 706B can be used to secure the distal end of the strap 700B. The fastener 706B can incorporate one of the following mechanisms including, but not limited to, a hook and loop system, Velcro, buttons, snaps, and/or magnets.

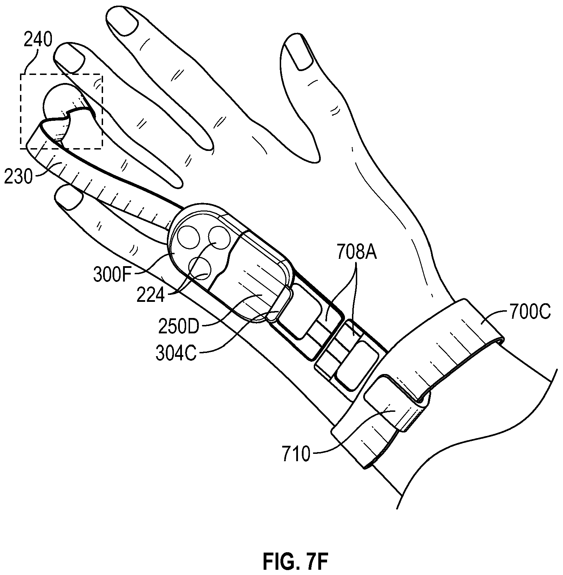

FIG. 7F shows yet another example of an attachment mechanism for the sensor assembly 202. The sensor assembly 202 can be coupled to an extender 708A which includes a hook 710. The extender 708A can extend away from the dock 222 of the sensor assembly 202, where the hook 710 is coupled to a distal end of the extender 708A. The hook 710 can wrap around the strap 700C such that the extender 708A and the dock 222 are substantially held in place with respect to a wrist of a patient. The strap 700C can be modular. The strap 700C may be removably connected or affixed to the hook 710 of the extender 708A. The strap 700C can be a flexible band that can tightly wrap around a patient's wrist, as shown in FIG. 7F.

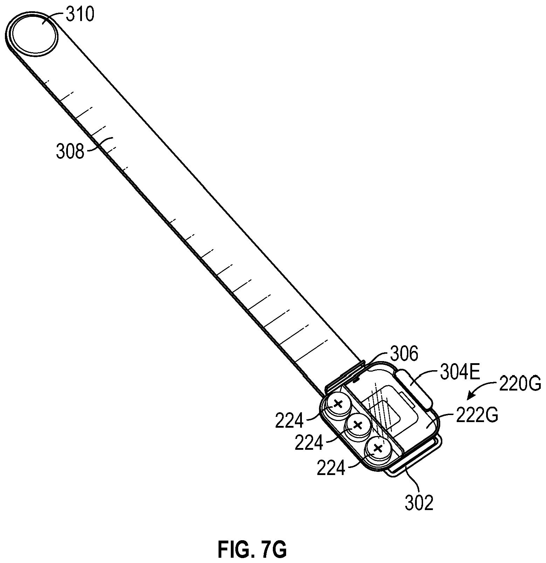

FIG. 7G shows yet another example of an attachment mechanism for the sensor assembly 202. The dock 222 can include the strap 308 extending from a first side of the dock 222, the strap 308 dimensioned to wrap around a patient's wrist in a first direction, and the strap loop 302 extending from a second side of the dock 222. The strap 308 can include the fastener 310 disposed near its distal end. The strap 3810 can be routed around the patient's wrist and through the strap loop 302 of the dock 222. Once routed through the strap loop 302 of the dock 222, the strap 308 can be routed around the strap loop 302 and wrap the wrist in a second direction. The first direction of wrapping the strap 308 around the wrist can be clockwise or counterclockwise. The second direction of wrapping the strap 308 around the wrist can be clockwise or counterclockwise. FIG. 7H shows the sensor assembly 202 of FIG. 3A affixed to a patient's wrist.

FIG. 7I illustrates yet another example of an attachment mechanism for the sensor assembly 202. The dock 222 and the sensor 240 can be coupled to a glove 712. When the glove 712 is placed on a patient's hand, the sensor 240 of the sensor assembly 202 can be placed one of the fingertips. The dock 222 can be attached to a top portion of the glove 712 as shown in FIG. 7I. The sensor 240 of the sensor assembly 202 can be built inside or outside the fingers of the glove 712. The sensor 240 can be integrated to the fingers of the glove 712. The cable 230 of the sensor assembly 202 can be integrated to the glove 712.

### Dongle and Pairing

Given the time demands placed on clinicians in busy hospitals and the number of patients and patient monitoring devices, manual interaction to establish connection between the computing device 206 (for example, a mobile patient monitoring display device) and the reusable module 250 can be burdensome. In some cases, the time required to manually interact with a patient monitor device in order to establish connection with a pairing device can even jeopardize a patient's well-being in particularly urgent circumstances. For at least the foregoing reasons, it would be advantageous for the computing device 206, such as bedside patient monitors, central monitoring stations, and other devices, to have the capability to detect the presence of the reusable module 250 nearby and establish a wireless communication 204 with the reusable module 250.

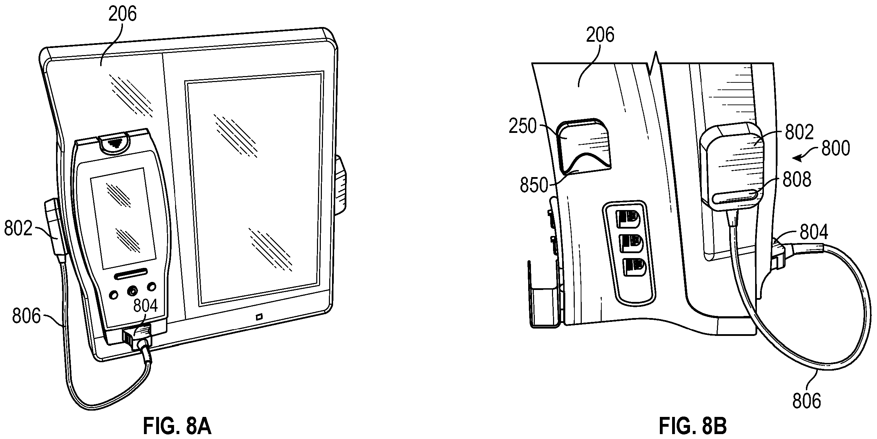

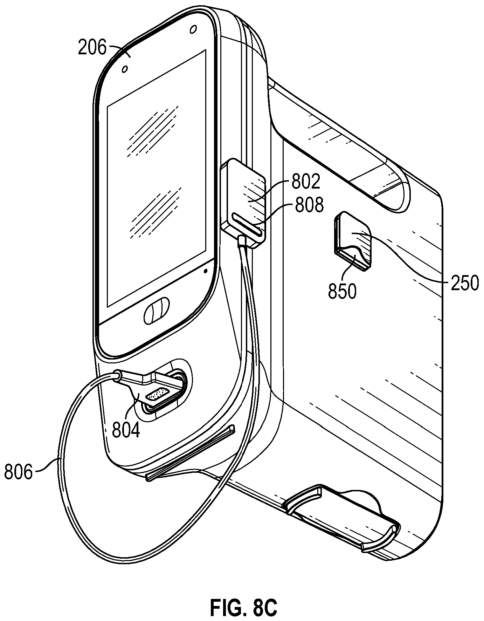

FIGs. 8A-8C illustrate various view of a dongle 800 connected to the computing device 206. The dongle 800 can include a body 802 and a connector 804 coupled to the body 802 via a cable 806. The connector 804 can connect to the computing device 206 to allow transmissions between the dongle 800 and the computing device 206. The cable 806 can include one or more conductive wires that can transmit data and/or power between the body 802 and the connector 804. The body 802 of the dongle 800 can be removably attached to the computing device 206. The body 802 can receive power from the computing device 206 via the connector 804 and the cable 806.

When the dongle 800 is connected to the computing device 206 via the connector 804, the computing device 206 can automatically detect the connector 804. The computing device 206 can determine a type of connector 804 and automatically change its settings. The settings may include, but not limited to, display settings for the display 208, display setting for the computing device 206 (for example, color of lights used to denote pair or communication status), communication protocol settings (for example, type of wireless communication utilized), communication signal settings (for example, varying communication signal type or strength based on different types of communications), and the like. Additionally, the settings for the dongle 800 can change to accommodate different types of computing devices 206 and their displays 208. Such setting can include display settings (for example, colors or messages denoting communication/pairing status), communication signal settings (for example, frequency of wireless signal used), communication protocol settings (for example, types of wireless communication used), and the like.

The computing device 206 can receive processed physiological parameter data and display on a display screen. This feature can be advantageous because it can reduce the amount of processing power required by the computing device 206. As discussed above, the reusable module 250 can perform signal processing on raw patient physiological data collected by the sensor 240 and calculate patient physiological parameters. Therefore, the data transmitted from the reusable module 250 to the computing device 206 via the body 802 includes patient physiological parameters that do not require further signal processing.

The reusable module 250 can transmit patient physiological parameters with low resolution and the dongle 800 can fill in the data using various methods. For example, the dongle 800 may use different types of averages to fill in the data transmitted from the reusable module 250. The reusable module 250 can send waveform data, for example, at a low resolution and the dongle 800 can increase the resolution of the waveform. This feature can further increase the life of the battery 224 of the disposable module 220.

The body 802 of the dongle 800 can include a transceiver or receiver, and a communication module for communicatively coupling the computing device 206 to other patient monitoring devices such as the reusable module 250. When the reusable module 250 is sufficiently proximate, the body 802 can communicate with the reusable module 250 so as to identify the reusable module 250. The body 802 can include a radio-frequency identification (RFID) reader and while the reusable module 250 can include an embedded RFID chip containing an identifying information unique to the reusable module 250. The RFID reader of the body 802 can identify the embedded RFID chip inside the reusable module 250 and establish a wireless communication 204 between the reusable module 250 and the body 802. The body 802 can include a transceiver that complies with one or more short-range wireless communications standards, such as Bluetooth^{®}. Other types of wireless communication protocols may be utilized to establish communication and transfer data between the dongle 800 and the reusable module 250.

The body 802 can include a groove 808 dimensioned to receive a portion of the reusable module 250. The groove 808 can indicate a medical personnel where to place the reusable module 250 in order to associate (for example, pair) the reusable module 250 with the computing device 206.

The dongle 800 can include a holder 850 that can retain the reusable module 250 when not in use. The holder 850 can be separate from the dongle 800 as shown in FIG. 8B. The holder 850 can include a surface dimensioned and shaped to engage with a surface of the reusable module 250 to assist in retaining the reusable module 250. The holder 850 can use a magnet to retain the reusable module 250. The holder 850 can be attached on the computing device 206 via various mechanisms including, but not limited to, adhesives, Velcro, magnet, and the like.

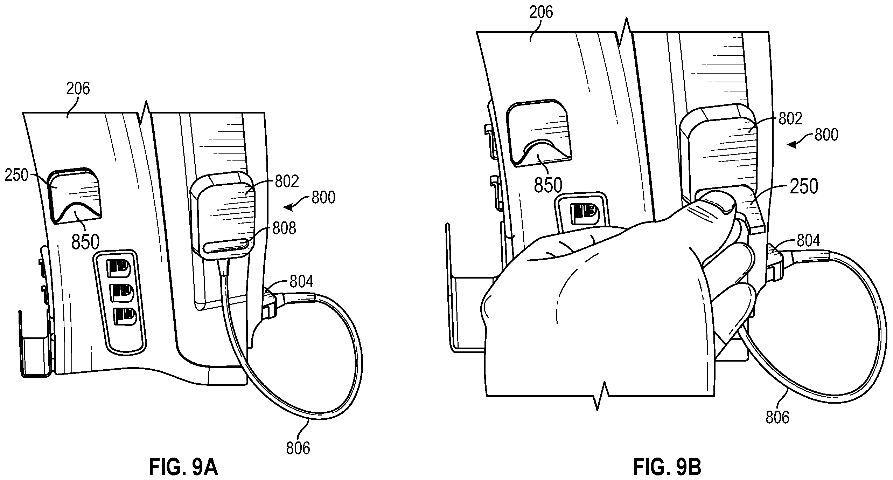

FIGs. 9A-9C illustrate a process of pairing the reusable module 250 with the computing device 206 using the dongle 800. Wireless communication 204 between the reusable module 250 and the computing device 206 can be initiated by coupling the connector 804 of the dongle 800 with the computing device 206 and placing the reusable module 250 within a certain distance away from the body 802 of the dongle 800. The reusable module 250 may or may not require a physical contact with the body 802 to transfer its identifying information to the dongle 800.

When the reusable module 250 is brought sufficiently close to the body 802 of the dongle 800, the body 802 can, for example, use RFID technology to receive from the reusable module 250 information that can identify the reusable module 250 to the computing device 206. The identifying information can be an ID tag of a token specific or unique to the reusable module 250. The identifying information can include Bluetooth^{®} parameters of the reusable module 250. Other types of identification mechanisms can be used to allow the computing device 206 to identify and associate with the reusable module 250.

The identifying information of the reusable module 250 can be stored in the memory 256. The identifying information may be hardwired into the memory 256 or programmable. The identifying information can include pairing parameters (for example, a pairing device ID) that is unique to the reusable module 250. The identifying information may be unique to the patient to whom the reusable module is assigned. The identifying information of the reusable module 250 may also include other information such as, for example, the pairing device's information, information regarding the sensor 240 the reusable module 250 is operatively connected to, or a code or other indicator for initiating a predetermined action to be performed by the computing device 206. Additionally and/or alternatively, the identifying information of the reusable module 250 can be generated using physiological data collected by the sensors 240 of the sensor assembly 202.

The body 802 of the dongle 800 can include a RFID reader. The RFID reader can communicatively couple the computing device 206 to other patient monitoring devices such as the reusable module 250. When the reusable module 250 is proximate to the body 802, as shown in FIG. 9B, the RFID reader of the body 802 can receive the identifying information from the reusable module 250. Once the body 802 receives the identifying information, the identifying information can be transmitted to the computing device 206 via the cable 806 and the connector 804.

The computing device 206 can use the identifying information to associate the reusable module 250 with the computing device 206. For example, the Bluetooth^{®} parameters of the reusable module 250 can be used to associate the reusable module with the computing device 206. Once associated, the reusable module 250 can connect with the computing device 206 using the pairing parameters (for example, Bluetooth^{®} parameters) included in the identifying information. The computing device 206 can identify the reusable module 250 and allow wireless communication 204 with the reusable module 250 using the Bluetooth^{®} parameters it received from the reusable module 250. After establishing connection with the computing device 206, the reusable module 250 can communicate with the dongle 800 and the computing device 206 via Bluetooth^{®} transmission. Other types or standards of wireless communication can be used, including, for example, ultrasound, Near Field Communication (NFC), and the like. If multiple reusable modules 250 are proximate to the computing device 206, a priority scheme or a user acknowledgment may be used to determine which reusable modules 250 are accommodated.

The reusable module 250 can use the NFC to provide instructions to program the dongle 800 to take certain actions in certain situations. The NFC communication circuitry of the reusable module 250 can have an associated memory that can have read/write capabilities. For example, the reusable module 250 can use NFC to indicate how long the dongle 206 must wait before deleting the pairing parameters ("giving up"). In another example, the reusable module 250 can use the NFC to indicate when the dongle 800 is disallowed from deleting the pairing parameters ("not giving up"). The NFC can be used to allow the dongle 800 to associate with one or more reusable modules 250 at the same time.

The dongle 800 can use the NFC to receive various types of information from the reusable module 250. The dongle 800 can receive information associated with NFC components of the reusable module 250 and determine sensor types, patient types, patient information, physician information, hospital information, authorized uses, authorized supplies, authorized manufacturers, emitter wavelengths, or indications of the usage or life of the reusable module 250, parameters the reusable module 250 is capable of measuring, and the like. For example, the dongle 800 can receive information via the NFC to determine that a particular reusable module 250 is designed to work with sensor assembly 202. The dongle 800 can also write back using NFC. For example, the dongle 800 can provide programming information through NFC to the reusable module 250. The dongle 800 can also write sensor usage information to the reusable module 250. For example, the reusable module 250 may only be allowed to be used a certain number of times before it must be discarded in order to maintain quality. This information can be written to the reusable module 250 through NFC communication.

Throughout the present disclosure, it is to be understood that the dongle 800 may be incorporated directly into the computing device 206. For example, the dongle 800 can be built into the circuitry of the computing device 206 such that the dongle 800 and the computing device 206 are in the same housing. In another example, the dongle 800 and the computing device 206 are in the same housing but the dongle 800 is not built into the circuitry of the computing device 206. The dongle 800 can be incorporated into the computing device 206 such that the dongle 800 is located near an outer housing or body of the computing device 206. Such a configuration can allow the reusable module 250 to readily establish wireless communication 204 with the dongle 800. The dongle 800 incorporated directly into the computing device 206 can prevent possible connection issues between the dongle 800 and the computing device 206.

Once the computing device 206 is associated with the reusable module 250, it can transmit a signal to the reusable module 250 indicating that the reusable module 250 is associated with the computing device 206. Different types of notifications can be generated when the reusable module 250 has successfully established wireless communication 204 with the computing device 206. The notifications can be generated by the computing device 206, the reusable module 250, or both.

The computing device 206 can provide an auditory notification or a visual notification on the display 208. For example, the computing device 206 can play a pattern of beeps or a predetermined melody for successful pairing. In another example, the computing device can play an auditory message such as "SpO₂ sensor number 1234 has been successfully paired with patient monitoring device A123." Visual notifications can include a blinking LED on the display 208. Another example of a visual notification can be in a form of text such as "Pairing successful" displayed on the display 208. The reusable module 250 has one or more LEDs to indicate status of wireless communication 204 with the computing device 206. For example, the reusable module 250 can include a red LED to indicate that no wireless communication 204 has been established between the reusable module 250 and the computing device 206. In another example, the reusable module 250 can include a blue LED to indicate that the reusable module 250 has established the wireless communication 204 with the computing device 206. A blinking green LED may be used to indicate that the computing device 206 is waiting for the reusable module 250 to establish the wireless communication 204 with the computing device 206. Different color LEDs and different schemes can be used to indicate different status of wireless communication 204 between the reusable module 250 and the computing device 206.

After receiving the pairing parameters from the reusable module 250, the computing device 206 can wait for a predetermined time period for the reusable module 250 to establish the wireless communication 204 (for example, Bluetooth^{®} connection). If the wireless communication 204 is not established within the predetermined time period, the pairing parameters can expire, requiring the reusable module 250 to retransmit the pairing parameters to the computing device 206 again. The predetermined time period can be modified.

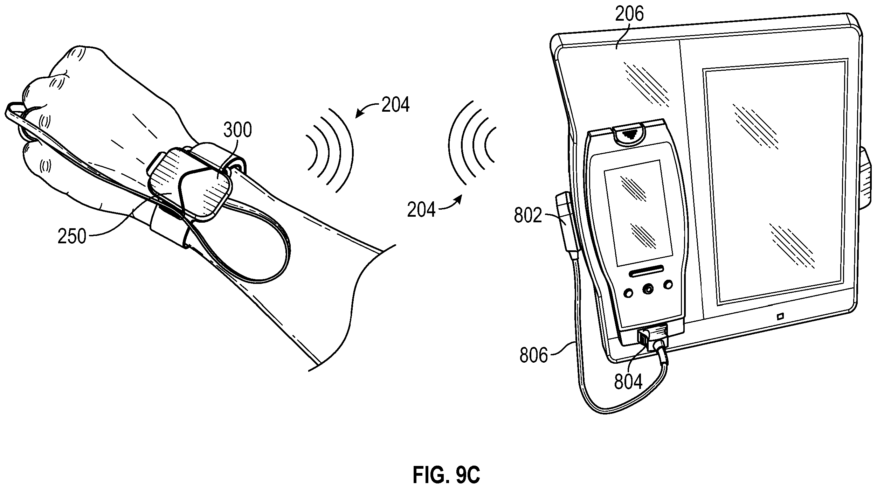

Once the computing device 206 receives the pairing parameters from the reusable module 250, the reusable module 250 can be mated with the dock 222, as shown in FIG. 9C. Once the reusable module 250 is mated with the dock 222, it can draw power from the battery 224 to establish wireless communication 204 with the computing device 206. The reusable module 250 can use the power drawn from the battery 224 to perform signal processing on the raw data to calculate physiological parameters. Once the physiological parameters are determined, the reusable module 250 can use the power from the battery to transmit the physiological parameters to the computing device 206 via the wireless communication 204.

The computing device 206 can receive the patient data including patient physiological parameters from the reusable module 250 and display the parameters on the display 208. The computing device 206 can receive the patient data via the body 802 of the dongle 800. In other words, the body 802 of the dongle 800 can receive patient physiological parameters from the reusable module 250 and in turn transmit the parameters to the computing device 206. As discussed above, Bluetooth^{®} can be used to transmit the patient data between the reusable module 250 and the computing device 206 (or the body 802). For example, the reusable module 250 operatively connected to a SpO₂ sensor can establish Bluetooth^{®} communication with the computing device 206. The computing device 206 can receive the patient data including SpO2 parameters from the reusable module 250 and display the parameters on the display 208. In another example, the reusable module 250 operatively connected to a temperature sensor can establish Bluetooth^{®} communication with the computing device 206. The computing device 206 can receive the patient data including temperature parameters from the reusable module 250 and display the parameters on the display 208. The computing device 206 can receive one or more parameters from the reusable modules 250 and display the one or more parameters on the display 208.

The reusable module 250 can include an ID tag that is active or passive RFID tag. An active RFID tag may be WiFi-enabled, for example. The ID tag can be a barcode (e.g., two-dimensional or three-dimensional) or a WiFi-enabled RFID tag. By communicating with the WiFi access points, the computing device 206 can triangulate its position relative to that WiFi access points. Likewise, the position of the reusable module 250 (and the sensor 240 if the reusable module 250 is operatively connected to the sensor 240) can be triangulated. Thus, the distributed WiFi access points can be used by, for example, the computing device 206 to determine the approximate position of the reusable module 250 (and/or the sensor 240) with respect to the computing device 206. The computing device 206 may also communicate directly with the reusable module 250 in order to, for example, enhance the position approximation determined using the distributed WiFi access points.

Positions of one or more reusable modules 250 can be used to determine relative or absolute positions of the one or more reusable modules 250. For example, consider reusable modules 250A, 250B, 250C, and 250D. When locations of the reusable modules 250A, 250B, and 250C are known, their positional information can be used to determine a position of the reusable module 250D.

The presence or proximity of the reusable module 250 to the computing device 206 may be determined by the reusable module 250 including an RFID tag. An "RFID tag" or simply "tag" can include any wireless communication device and/or communication standard (e.g., RFID, NFC, Bluetooth, ultrasound, infrared, and the like) that can remotely identify a proximate user to a monitor. Tags include, but are not limited to, devices in the form of badges, tags, clip-ons, bracelets or pens that house an RFID chip or other wireless communication components. Tags also encompass smart phones, PDAs, pocket PCs and other mobile computing devices having wireless communications capability. The RFID tag can include identifying information or pairing parameters for the reusable module 250.

The computing device 206 may respond to the departure of all proximate reusable modules 250 by automatically removing displays associated with the reusable modules 250. This feature can provide display patient physiological data only for sensors 240 associated with reusable modules 250 proximate to the computing device 206. The computing device 206 may respond in a similar manner by automatically silencing pulse "beeps" or other non-critical sounds when there are no proximate reusable modules 250 and associated sensors 240.

The computing device 206 can generate alarms when its wireless communication 204 with the reusable module 250 is disrupted or no longer exists. For example, the computing device 206 can create at least one of auditory and visual alarm when the reusable module 250 is no longer mated with the disposable sensor 220.

The computing device 206 can monitor signal strength of the wireless communication 204 between the computing device 206 and the reusable module 250. Under some circumstances, the reusable module 250 may move out of the range of the computing device 206 which may cause the wireless communication 204 to be disrupted. For example, a patient equipped with the reusable module 250 may visit an x-ray room for a routine visit and disrupt the wireless communication 204 between the reusable module 250 and the computing device 206. If the same reusable module 250 becomes available within the range within a period of time, the computing device 206 can automatically reestablish the wireless communication 204. For example, if the patent returns from the x-ray room within 30 minutes, the computing device 206 may be able to reestablish the wireless communication between the reusable module 250 and the computing device 206. Upon reestablishing communications, any information stored on the reusable module 250 for the time period where communication was disrupted can be downloaded to the computing device 206.

The computing device 206 can be configured to not lose (or delete) the pairing parameters received from the reusable dongle 250. This feature can prevent other reusable modules 250 from pairing with the computing device 206 even when the reusable module 250 is no longer wirelessly communicating with the computing device 206. For example, a first computing device 206 and a first reusable module 250 are in a first wireless communication 204. The first computing device 206 can be configured to not "give up" or "give up" the first reusable module 250 even after the first wireless communication 204 is terminated. When configured to "give up," a second reusable module 250 can be paired with the first computing device 206. When configured to "not give up," a second reusable module 250 cannot be paired with the first computing device 206.

This feature can also apply in situations in which the battery 224 of the disposable module 220 is about to be depleted or when the reusable module 250 is removed from the disposable module 220. Without power from the battery 224, the reusable module 250 cannot maintain the wireless communication 204 with the computing device 206. The computing device 206 can be configured to prevent or not prevent other computing device 206 from establishing wireless communication 204 with the reusable module 250. The reusable module 250 can also send a "dying" signal to the computing device 206 providing instructions on pairing or other instructions as the device is removed from the disposable module 220 or when the batteries are depleted. This dying instruction allows the pairing to be maintained.

Computing devices 206 (or dongle 800) can communicate to other computing devices 206 (or other dongles 800) to ensure that each computing device 206 (or dongle 800) is paired to a single reusable module 250 at any time. For example, when a first reusable module 250 is paired (or associated) with a first computing device 206, a second reusable module 250 may not be paired (or associated) with the first computing device 206. However, the first reusable module 250 may be able to pair with a second computing device 206. Pairing the first reusable module 250 with the second computing device 206 can cause the second computing device 206 to inform the first computing device 206 to release its pairing with the first reusable module 250.

The computing device 206 can identify the sensors 240 and the reusable modules 250 associated with the computing device 206. When one or more sensors 240 and reusable modules 250 are wirelessly associated to the computing device 206, it may be advantageous for the computing device 206 to distinguish and indicate different physiological parameters from different sensors 240 or reusable devices 250. For example, the computing device 206 can be associated with two different sensors 240 (and their respective reusable modules 250) for detecting peripheral capillary oxygen saturation (SpO₂) and acoustic respiration rate (RRa). The computing device 206 can display information pertaining to the sensors 240 or the reusable modules 250 (for example, sensor name, sensor type, sensor location, sensor ID, reusable module ID, reusable module name) to distinguish patient parameters from different sensors and/or reusable modules.

The reusable module 250 of the sensor assembly 202 can establish wireless communication 204 with mobile devices such as smartphones, tablets, smartwatches, laptops, and the like. The mobile devices can include a mobile application that allows the mobile devices to establish wireless communication 204 with the reusable module 250 of the sensor assembly 202, receive patient physiological parameters from the reusable module 250, and display the patient physiological parameters. In addition to the patient physiological parameters, the mobile application can also display other patient information including, but not limited to, name, age, past medical history, current medications, address, gender, and the like.

The wireless communication 204 between the mobile devices and the reusable module 250 can be in a form of Bluetooth^{®}. The wireless communication 204 between the mobile devices and the reusable module 250 can be established via the Internet. For example, the computing device 206 can be connected to the Internet or a secured network server. Once wireless communication 204 between the reusable module 250 and the computing device 206 is established, the mobile devices can access the Internet or the secure network server to receive and display the patient physiological parameters via the mobile application described above.

The mobile application can include various security measures to prevent third-parties from accessing patient information. The mobile application can be associated with certain mobile devices that has been identified by a healthcare provider. Identification and a passcode may be required for using the application to connect to the reusable module 250 (or the computing device 206), receive patient data (for example, patient data and/or patient physiological parameters), and display patient data. Each of the mobile applications can be associated with a unique access code or an identification code that may be required for receiving patient data from the Internet or the secured network server. The unique access code or the identification code can be associated with the mobile device or the mobile application. The unique access code can be a media access control (MAC) address associated with each of the mobile devices.

### Mating of the Dock and Reusable Module

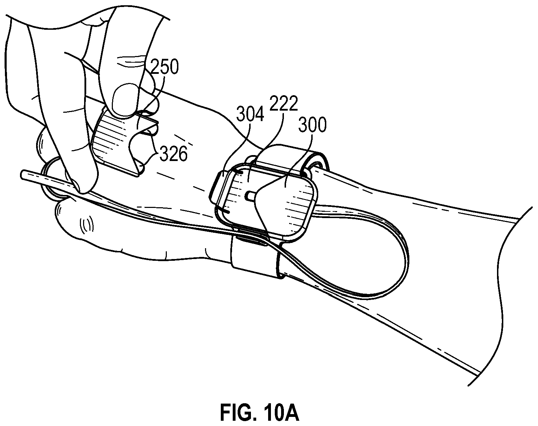

FIGs. 10A-10D illustrates the process of mating the reusable module 250 with the dock 222 of the disposable module 220. The dock 222 of the disposable module 220 can be attached to a wrist of a patient as shown in FIG. 10A. The dock 222 can include a housing 300 that includes slots 328 (see FIG. 3B) that correspond to the legs 326 of the reusable module 250.

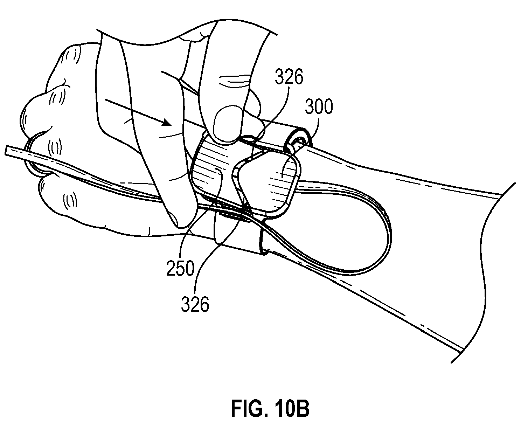

FIG. 10B illustrates the reusable module 250 being inserted into the dock 222. The legs 326 can face the slots 328 of the dock 222 as the reusable module 250 is inserted. When the legs 326 are substantially positioned within the slots 328 of the dock 222, body of the reusable module 250 can be positioned at an angle with respect to the dock 222. One end of the reusable module 250 may be positioned on top of the retainer 304 while at least a portion of the legs 326 are positioned in the slots 328 of the dock 222.

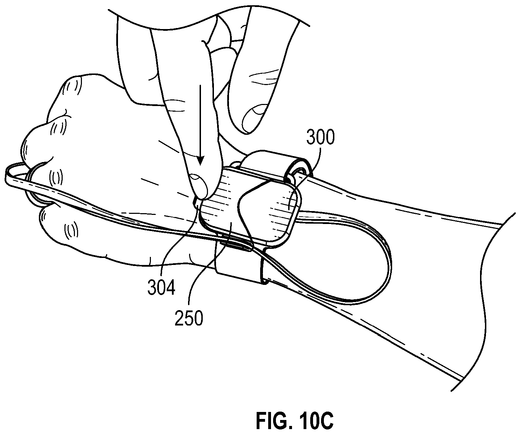

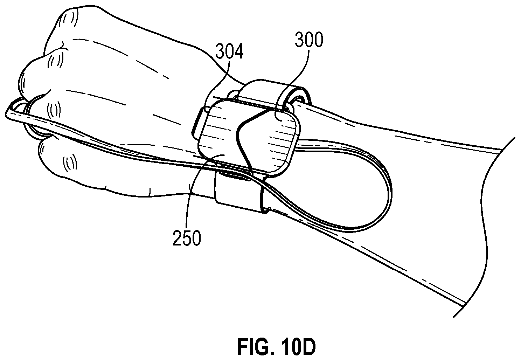

FIG. 10C illustrates the reusable module 250 being pushed down towards the dock 222. As shown in the FIG. 10C, the legs 326 can be partially inserted in the slots 328. The reusable module 250 can be pushed down, which causes the retainer 304 to move away from the housing 300, thus allowing the reusable module 250 to be fully inserted in the dock 222 and mated with the dock 222 as shown in FIG. 10D. When the reusable module 250 is fully inserted, the retainer 304 can snap back in a direction towards the housing 300 and engage with the groove 322 of the reusable module 250 (FIG. 3B). Mating between the reusable module 250 and the dock 222 can cause the legs 326 engage the slots 328 of the housing 300. The engagement between the groove 322 and the protrusion 324 (FIG. 3B) of the retainer 304 can hold the reusable module 250 in place while mated with the dock 222. The engagement between the slots 328 and the legs 326 can hold the reusable module 250 in place.

### Methods of Pairing, Collecting Data, and Transmitting Data to Computing Device

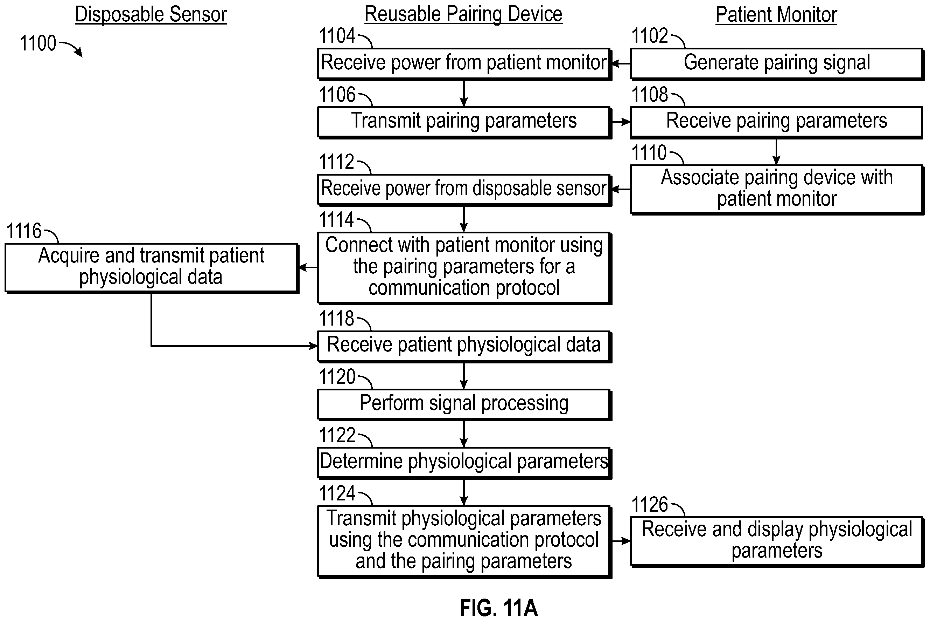

FIG. 11A illustrates a method 1100 of establishing wireless communication between the reusable module 250 and the computing device 206, determining patient physiological parameters using the sensor assembly 202, and displaying the physiological parameters using the computing device 206.

At block 1102, a patient monitor (for example, the computing device 206) can generate and transmit a pairing signal. Generating the transmitting the pairing signal can be done automatically or manually. The pairing signal may be a radio signal. The pairing signal can be configured such that a nearby device, upon receiving the signal, is triggered to transmit an identification information in response. The nearby device may be the reusable module 250. The pairing signal can also contain sufficient power to enable nearby devices to transmit pairing parameters in response to the pairing signal.

Generating and transmitting the pairing signal can be done by different devices. The computing device 206 can generate the pairing signal while the dongle 800 attached to the computing device 206 via the connector 804 can transmit the pairing signal. The dongle 800 can generate and transmit the pairing signal for the computing device 206.

The reusable module 250 located within a predetermined distance from the computing device 206 can receive the pairing signal. This can be advantageous in hospital environments where many patients can be placed within a short distance from an electronic device such as the computing device 206. Such configuration can allow the electronic device (for example, the computing device 206) to receive patient health data only from a patient who is nearby and prevent the electronic device from receiving patient health data from other patients who may not be a patient-in-interest. Strength of the pairing signal can be varied to allow the signal to travel further or closer.

At block 1104, the reusable module 250 can receive power from the pairing signal generated by the computing device 206. The pairing signal can be a high-frequency alternating current which can be used to create a voltage potential. The pairing signal of the computing device 206 may be received when the reusable module 250 is within a predetermined distance. As discussed above, physical contact between the computing device 206 (or the dongle 800) and the reusable module 250 may be required for the reusable module 250 to receive the power from the pairing signal. The reusable module 250 can automatically receive power from the pairing signal. By receiving power from the pairing signal, the antenna 252 of the reusable module may not need to draw power from the battery 224 of the disposable device 220.

At block 1106, the reusable module 250 can use the power received from the pairing signal to transmit identification information to the computing device 206. The identification information can include pairing parameters of the reusable module 250. The identification information may be a tag serial number unique to the reusable module 250. The identification information can include, but not limited to, stock number, lot number, batch number, production date, or other specific information. The computing device 206 can use the identification information to uniquely identify the reusable module 206. The transmission of the identification information can occur automatically.

The reusable module 250 can include a feature that prevents automatic transmission of the identification information to the computing device 206. This feature can be advantageous to prevent inadvertent pairing of the reusable module 205 with the computing device 206. Medical personnel can deal with patients in need of many different types of sensors. In such circumstances, reusable modules 250 may inadvertently be brought proximal to the computing device 206 (or dongle 800). Thus it can be advantageous for the reusable module 250 to have the feature to prevent the reusable modules 250 from automatically pairing with the computing device 206 (or dongle 800) to prevent inadvertent pairing.

At block 1108, the computing device 206 can receive the identification information from the reusable module 250. The dongle 800 connected to the computing device 206 can receive the identification information and relay it to the computing device 206. At block 1110, the computing device 206 can associate with the reusable module 250, which allows the wireless communication 204 to be established between the reusable module 250 and the computing device 206.

The association between the computing device 206 and the reusable module 250 can occur automatically. On the other hand, the association can require a user input via the computing device 206. For example, upon receiving the pairing parameters from the reusable module 250, the computing device 206 can generate a notification prompting a user to allow or disallow the computing device 206 to associate with the reusable module 250. If allowed, the computing device 206 can associate with the reusable module 250 and the reusable module 250 can establish a wireless communication 204 with the computing device 206. If not allowed, the computing device 206 may not associate with the reusable module 250 and the reusable module 250 may not establish a wireless communication 204 with the computing device 206.

Establishing wireless communication 204 can require the reusable module 250 to have an external power source. The battery 224 provides sufficient power for the reusable module 250 to receive raw patient physiological data from the sensor 240 and perform signal processing on the raw data to calculate patient physiological parameters. Moreover, the reusable module 250 can use the power from the battery 224 to use the antenna 252 to wirelessly transmit the calculated parameters to the computing device 206. Without the battery 224 connected to the dock 222, the reusable module 250 cannot receive power via the electrical contacts 228, 258.

At block 1112, the reusable module 250 can mate with the dock 222 and receives power from the battery 224 via the battery circuit 314 and the electrical contacts 228, 258. At block 1114, the reusable module 250 can establish wireless communication 204 with the computing device 206. The wireless communication 204 can be established using the pairing parameters. The wireless communication 204 can be via Bluetooth^{®}, as discussed above. The wireless communication 204 can be one-way or two-way communication between the reusable module 250 and the computing device 206. For example, the reusable module 250 can transmit calculated physiological parameters to the computing device 206. The computing device 206, in return, can transmit a confirmation signal back to the reusable module 250 to let the reusable module 250 know that the calculated parameters were received. The reusable module 250 can include one or more light sources (for example, LEDs) that can generate light when the reusable module 250 receives the confirmation signal from the computing device 206.

At block 1116, the sensor 240 can acquire raw patient physiological data and transmits the data to the dock 222 via the cable 230 and the flex circuit 320. The raw physiological data can be transferred to the reusable module 250 via the electrical contacts 228, 258. The sensor 240 can include, but not limited to, an acoustic sensor, ECG sensor, EEG sensor, respiratory acoustic sensor (RAS), SpO₂ sensor, and the like. The sensor 240 can include one or more different types of sensors.

The sensor 240 can be placed on various areas of a patient. The location of the sensor 240 can depend on the type of sensor used for the sensor 240. For example, the sensor 240 can be an O₃ sensor typically adhered to a patient's forehead to monitor cerebral oxygenation. In another example, the sensor 240 can be a respiratory acoustic sensor typically attached to a patient's neck near the trachea to detect vibrations associated with respiration.

At block 1118, the processor 254 of the reusable module 250 can receive the raw patient physiological data from the sensor 240 of the disposable module 220. The raw patient physiological data can be stored in the memory 256.

At block 1120, the processor 254 of the reusable module 250 can perform signal processing on the raw physiological data. Various types of signal processing used on the physiological data raw can include, but not limited to, analog signal processing, continuous-time signal processing, discrete-time signal processing, digital signal processing, or nonlinear signal processing. For example, continuous-time signal processing such as time domain, frequency domain, and complex frequency domain can be used. Some of the signal processing methods that can be used on the raw physiological data include, but not limited to, passive filters, active filters, additive mixers, integrators, delay lines, compandors, multiplicators, voltage controlled filters, voltage controlled oscillators, phase-locked loops, time domain, frequency domain, fast Fourier transform (FFT), finite impulse response (FIR) filter, infinite impulse response (IIR) filter, and adaptive filters. Such processing techniques can be used to improve signal transmission, storage efficiency, and subjective quality. In addition, such processing techniques can be used to emphasize or detect components of interest in the raw physiological data. Noise filtering can be used to filter out raw physiological data corrupted by noise due to patient movement, electromagnetic interference, or ambient light.

Signal processing can determine the absorbance's of the light due to pulsating arterial blood. For example, pulse oximeter generates a blood-volume plethysmograph waveform from which oxygen saturation of arterial blood, pulse rate, and perfusion index, among other physiological parameters, can be determined. In the context of pulse oximetry, the sensor 240 can use adaptive filter technology to separate an arterial signal, detected by a pulse oximeter sensor, from the non-arterial noise for example, venous blood movement during motion). During routine patient motions (shivering, waving, tapping, etc.), the resulting noise can be quite substantial and can easily overwhelm a conventional ratio based oximetry system. This can provide accurate blood oxygenation measurements even during patient motion, low perfusion, intense ambient light, and electrocautery interference.

At block 1122, the processor 254 of the reusable module 250 can determine patient physiological parameters by processing the raw physiological data. The processor 254 can then store the processed data and the calculated parameters in the memory 256 before transmitting them to the computing device 206.

The processed data can be indicative of an amount of attenuation of predetermined wavelengths (ranges of wavelengths) of light by body tissues, such as, for example, a digit, portions of the nose or year, a foot, or the like. For example, the predetermined wavelengths correspond to specific physiological parameter data desired including, but not limited, blood oxygen information such as oxygen content (SpOC^{®}), oxygen saturation (SpO₂), blood glucose, total hemoglobin (SbHb), methemoglobin (SpMet^{®}), carboxyhemoglobin (SpCO), bulk tissue property measurements, water content, pH, blood pressure, respiration related information, cardiac information, perfusion index (PI), pleth variability indices (PVI^{®}), or the like, which can be used by the mobile computing device to determine the condition of the user. The processed data can provide information regarding physiological parameters such as EEG, ECG, heart beats per minute, acoustic respiration rate (RRa), breaths per minute, end-tidal carbon dioxide (EtCO₂), respiratory effort index, return of spontaneous circulation (ROSC), or the like, which can be used to determine the physiological condition of the user.

At block 1124, the processor 254 of the reusable module 250 can transmit the patient physiological parameters to the computing device 206 via the antenna 252 using the communication protocol and the pairing parameters. It can be advantageous to transmit the calculated physiological parameters (for example, 60% SpO₂) as opposed to transmit the raw physiological data to the computing device 206. Compared to calculated physiological parameters, the raw physiological data can be larger in size and thus require larger bandwidth during transmission to the computing device 206. Calculated physiological parameters, on the other hand, can be much smaller in size and can require smaller bandwidth to transmit. Therefore, transmitting patient physiological parameters instead of raw physiological data can lead to decreased battery consumption and longer battery life for the disposable module 220.

The transmission of the physiological parameters can occur wirelessly via NFC. For example, the transmission of the physiological parameters occur wirelessly via Bluetooth. The transmission of the physiological parameters may occur via a cable.

At block 1126, the computing device 206 can receive the patient physiological parameters and displays them using the display 208. As discussed above, the computing device can include the display 208 that can display various patient physiological parameters including, but not limited to, body temperature, heart rate, blood oxygen level, blood pressure, and the like.

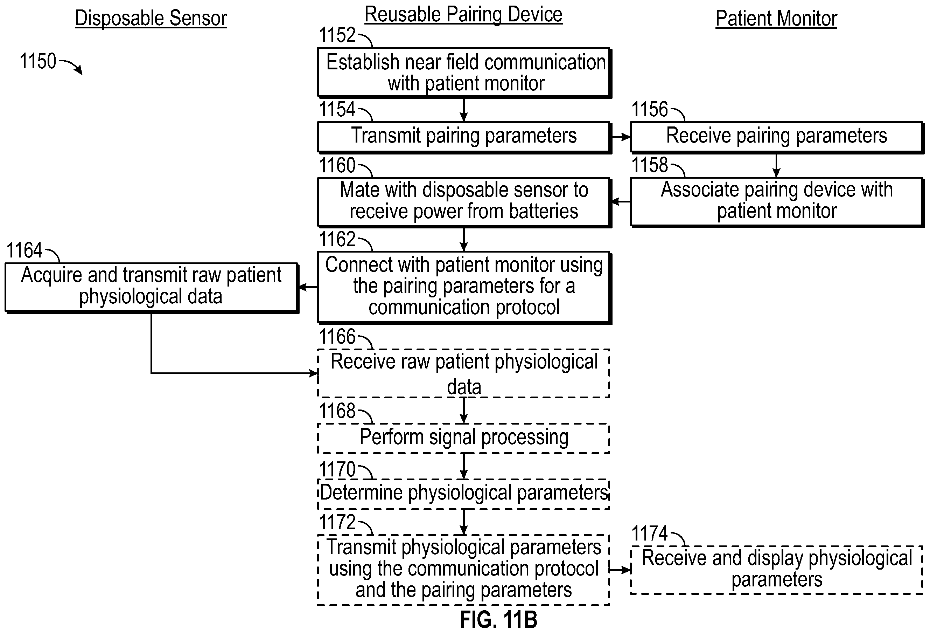

FIG. 11B illustrates another method 1150 of establishing wireless communication between the reusable module 250 and the computing device 206, determining patient physiological parameters using the sensor assembly 202, and displaying the physiological parameters using the computing device 206.

At block 1152, the reusable module 250 can establish a NFC (near field communication) with the computing device 206. As discussed above, establishing a NFC can require the reusable module 250 to be within a predetermined distance of the computing device 206. As noted above, the NFC can be established between the body 802 of the dongle 800 and the reusable module 250.

At block 1154, the reusable module 250 can transmit pairing parameters to the computing device 206. The transmission of the pairing parameters to the computing device 206 can occur when the reusable module 250 establishes the NFC with the computing device 206. At block 1156, the computing device 206 can receive the pairing parameters from the reusable module 250. The computing device 206 can use the dongle 800 to receive the pairing parameters. For example, the body 802 of the dongle 800 can wirelessly receive the pairing parameters and transmit the pairing parameters to the computing device 206 via the cable 806 and the connector 804.

At block 1158, the computing device 206 or the body 802 can associate with the reusable module 250 using the pairing parameters. Once associated, the computing device 206 or the body 802 may wait for the wireless communication 204 from the reusable module 250. As noted above, the wireless communication 204 can be made via Bluetooth^{®}. At block 1164, the sensor 240 of the disposable module 220 can acquire physiological data and transmit the data to the reusable module 250. The physiological data acquired by the sensor 240 and transmitted to the reusable module 250 can be raw physiological data.

Blocks 1166 through 1174 may be optional. At block 1166, the reusable module can receive the patient physiological data from the disposable module 220. At block 1168, the reusable module 250 can perform signal processing on the patient physiological data. At block 1170, the reusable module 250 can determine patient physiological parameters using the processed physiological data. At block 1172, the reusable module 250 can transmit patient physiological parameters using the wireless communication 204 established between the reusable module 250 and the computing device 206. The body 802 of the dongle 800 may wirelessly receive the patient physiological parameters from the reusable module 250 and transmit the parameters to the computing device via the cable 806 and the connector 804. At block 1174, the computing device 206 receives the patient physiological parameters and displays the parameters on the display 208.

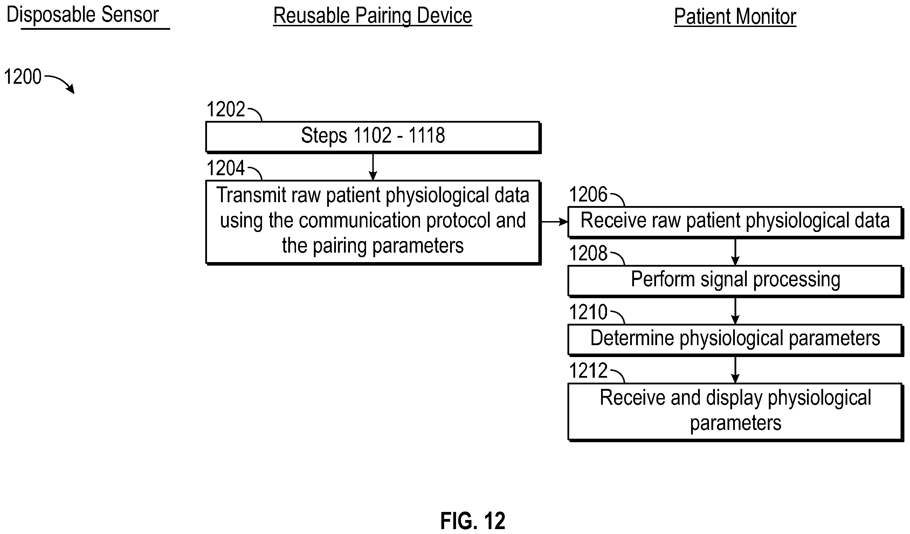

FIG. 12 illustrates another method 1200 of determining patient physiological parameters using the sensor assembly 202 and displaying the physiological parameters using the computing device 206.

At block 1202, the processor 254 of the reusable module 250 receives raw patient physiological data from the sensor 240 of the disposable module 220 according to the blocks 1102-1120 of FIG. 11.

At block 1204, the processor 254 of the reusable module 250 transmits the raw patient physiological data to the computing device 206. The process 254 can use the antenna 252 to transmit the raw data via the wireless communication 204 established between the reusable module 250 and the computing device 206. As mentioned above, the wireless communication 204 can be one-way or two-way between the reusable module 250 and the computing device 206.

At block 1206, the computing device 206 receives the raw patient physiological data. At block 1208, the computing device 206 performs signal processing on the raw patient physiological data. At block 1210, the computing device 206 determines patient physiological parameters using processed raw patient physiological data. At block 1212, the computing device 206 displays the determined physiological parameters on the display 208.

### Mobile Application

As discussed above, the computing device 206 can be a mobile device 1300 such as a phone, tablet, watch and the like. The mobile device 1300 can include a mobile application that can establish wireless communication with the reusable module 250 via a wireless communication protocol, such as Bluetooth or the like.

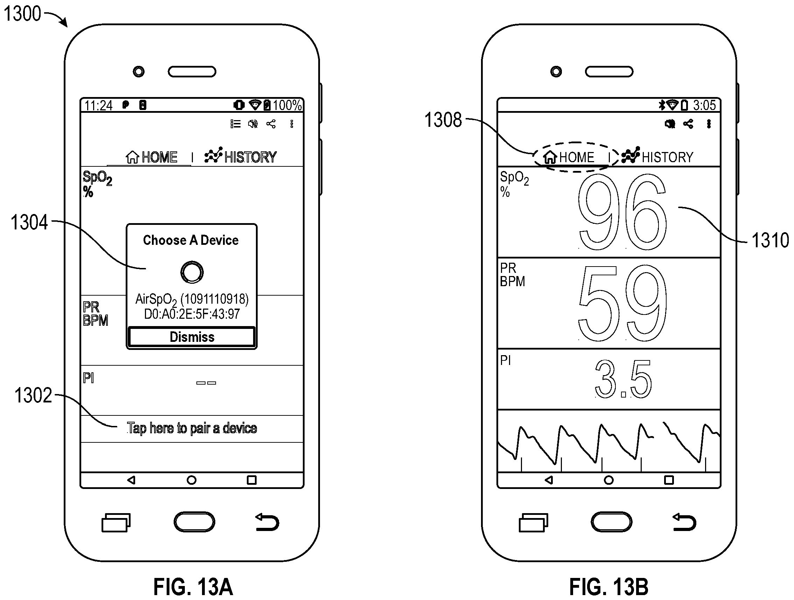

FIG. 13A illustrates a mobile application being executed on the mobile device 1300 (for example, a mobile phone) to establish a wireless communication with the reusable module 250. The mobile application can pair with nearby reusable modules 250. In an example, a user can press a pair button 1302 to cause the mobile application to search for nearby reusable modules 250. The mobile application can create a screen 1304 to display nearby reusable modules 250. The screen 1304 can provide MAC address or any other pairing information unique to the reusable modules 250. The mobile application may automatically search for nearby reusable modules 250 without any user intervention or input.

FIGs. 13B-13E illustrate various examples the mobile application displaying patient parameters. Triggering a home button 1308 can cause the mobile application to show real-time, numerical and graphical illustration of patient parameters, as shown in FIG. 13A. The mobile application can show numerical parameters 1310 (for example, patient's SpO₂, PR BPM, and PI readings) in real time or with a predetermined delay. The mobile application may show graphical illustration 1314 of patient parameters that show real-time trend of the parameters. For example, a user can trigger an SpO₂ portion of the display to cause the mobile application to show real-tine trend of the SpO₂ parameters.

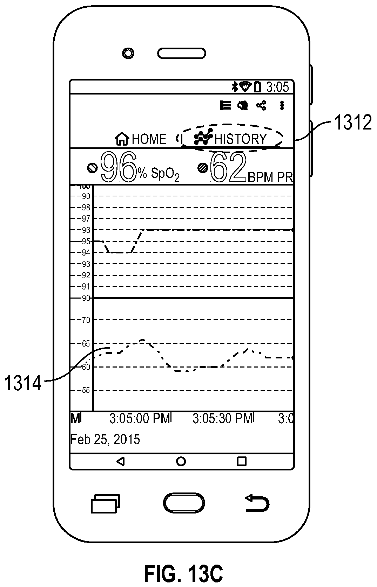

As shown in FIG. 13C, triggering a history button 1312 can cause the mobile application to show the graphical illustration 1314 showing historical trends of patient health parameters. The graphical illustration 1314 can have an x-axis showing timestamp and a y-axis showing parameter values. The mobile application may show real-time numerical values of patient health parameter above or below the graphical illustration 1314. The real-time numerical values can be embedded within the graphical illustration 1314.

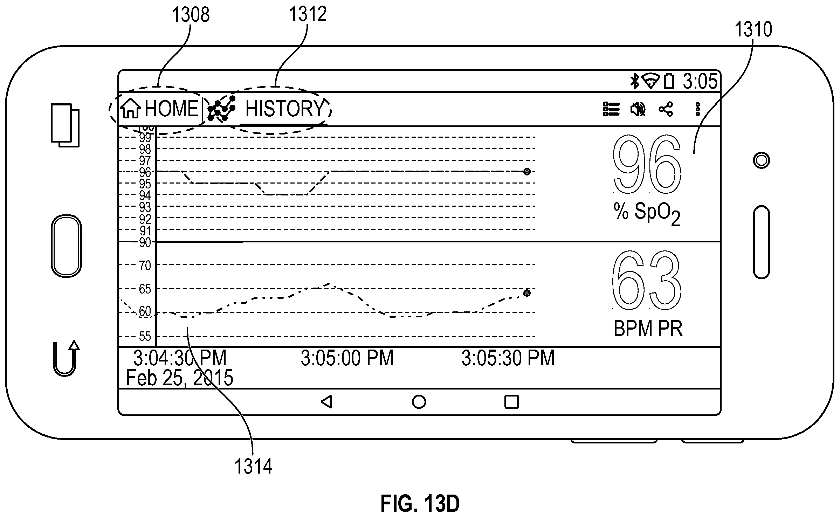

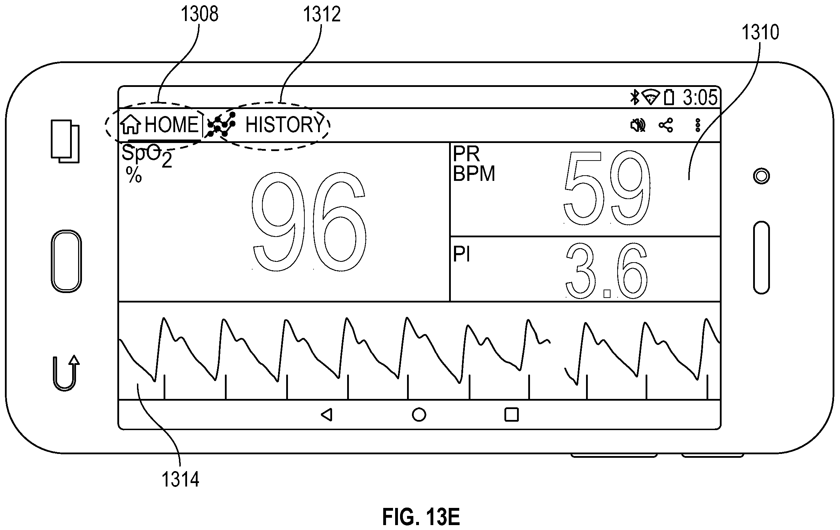

As shown in FIGs. 13D and 13E, the mobile application can display at least one of the numerical parameters 1310 and the graphical illustration 1314 in a landscape view.

### Methods of Identifying and/or Validating Disposable Module

As discussed herein, the disposable module 220 can include a sensor assembly 240 that can include various types of sensors. Accordingly, it may be advantageous for the reusable module 250 to be able to identify the disposable module 220. This can be advantageous, for example, to check or ensure that the sensor assembly 240 of the disposable module 220 has a desired or correct type of sensor suitable for certain situations, circumstance, and the like. In addition, the reusable module 250 can also obtain identification and/or operating parameters to ensure that the reusable module 250 uses a correct algorithm or calibration curve for the attached disposable sensor.

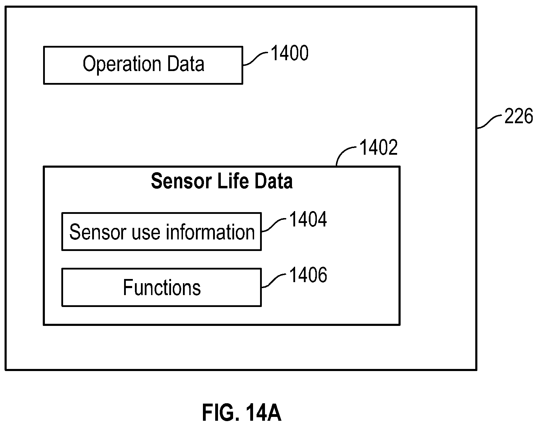

A memory of the disposable module 220, for example, the memory 226 shown in Figure 2B, can be configured to store operation data 1400 as shown in Figure 14A. The operation data 1400 may or may not be unique to the disposable module 220. The operation data may indicate a type of sensor, or types of sensors, associated with a given disposable module 220 at any given time. For example, the operation data 1400 may automatically be updated when a new sensor 240 is provided for the disposable module 220. Accordingly, the operation data 1400 can accurately reflect what sensors 240 are associated with a given disposable module 220 and provide such information to care providers. This can be useful in situations in which different types of sensors may look similar or when care providers do not have sufficient amount of time to check and ensure all sensors are properly identified.

Optionally, a reusable module, for example, the reusable module 250 shown in Figure 2B, may be programmed to be associated with specific types of sensors or specific types of patient health data. For example, the reusable module may be programmed to be associated with patient health data such as, for example, pulse oximetry related data, including, but not limited to, blood oxygen saturation level (SpO₂). As such, the reusable module may, when connected with a disposable module, access and analyze operation data of the disposable module to ensure that sensor assembly associated with or of the disposable module is, for example, compatible with the reusable module, or, for example, capable of collecting patient health data associated with blood oxygen saturation level.

The memory of the disposable module 220 may include sensor life data 1402. The sensor life data 1402 that may be used to monitor, for example, life expectancy of the disposable module 220. The sensor life data 1402 can include one or more sensor use information 1404 and one or more functions 1408 that can be used to determine sensor life expectancy. The sensor life expectancy can represent expected operation time of the disposable module 220. Some examples of sensor use information 1404 may include, but are not limited to, an age of the sensor, a user time of the sensor, a current supplied to the sensor, a temperature of the sensor, a number of times a sensor is depressed, a number of times the sensor is calibrated, a number of times the sensor is powered up, and the like. Various examples of systems and methods of determining sensor life expectancy using the sensor life data, for example, sensor use information and functions, is disclosed in U.S. Application No. 11/580,214, filed October 12, 2006, entitled "SYSTEM AND METHOD FOR MONITORING THE LIFE OF A PHYSIOLOGICAL SENSOR," now U.S. Patent No. 7,880,626, issued February 1, 2011. In some configurations, the life of the sensor is the life of the battery included on the sensor. When the battery is exhausted, the sensor records an end of life event on the sensor memory.

According to the present invention, the sensor life expectancy is automatically updated when there is a change in patient condition or a change in operation condition for the disposable module 220. For example, based at least in part on physiological data collected by the disposable module 220, a change in patient condition, for example, a sudden increase in blood pressure and decrease in blood oxygen level, may be identified. As described herein, detecting such change in patient condition may trigger the disposable module 220 to collect physiological data, for example, more frequently or at a higher fidelity, which may cause increased power consumption by the disposable module 220. In another example, the temperature of the disposable module 220 (and its sensor element) may increase and the sensor life expectancy may be automatically updated when the increase in the temperature of the disposable module 220 is detected. By automatically calculating and updating the sensor life expectancy under such example conditions described herein, care providers may have access to more accurate forecast of sensor life expectancy. This can be advantageous in situations in which, for example, a patient may be experiencing an emergency situation and the disposable sensor is having to, for example, collect more data points at higher fidelity. By automatically updating sensor life expectancy, the care providers may accurately monitor predicted operation time of the disposable module 220 and determine whether additional disposable module(s) 220 may be needed.

In some implementations, the sensor life expectancy may be automatically updated at a predetermine time interval. The predetermined time interval for updating the sensor life expectancy may range between about 1 minute and about 1 hour, between about 2 minutes and about 30 minutes, between about 5 minutes and about 20 minutes, between about 10 minutes and about 15 minutes, or about 1 minute, about 2 minutes, about 5 minutes, about 10 minutes, about 15 minutes, about 20 minutes, about 30 minutes, about 1 hours, or range between any two of aforementioned values.

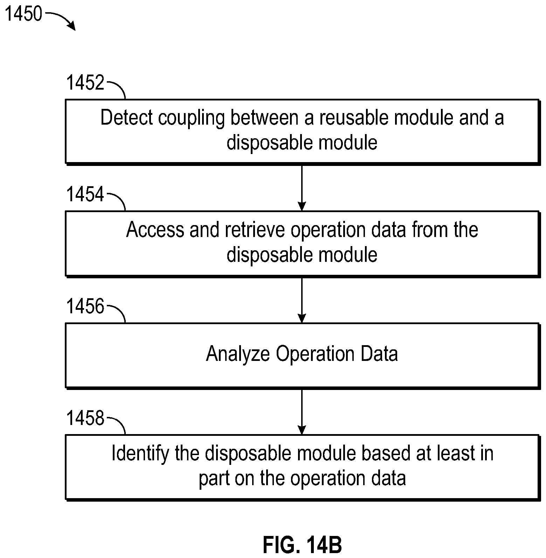

Figure 14B illustrates an example method of 1450 of identifying or validating a disposable module. At block 1452, coupling between a disposable module and a reusable module is detected. A processor, for example the processor 254 of the reusable module 250, of a reusable module may detect coupling between the reusable module and a disposable module by detecting an electronic input or signal transmitted between the reusable module and the disposable module. The electronic input or signal can be patient health data and/or current flowing from a battery, for example, the battery 224 of the disposable module 220, to the reusable module, and the like. Optionally, the computing system 206 can detect coupling between a reusable module and a disposable module by, for example, detecting patient health data and/or parameters transmitted from the reusable module 250.

At block 1454, operation data is accessed from the disposable module 220. As discussed herein, the operation data can be stored within a memory, for example, the memory 226, of the disposable module 220. In some examples, the processor, for example, the processor 254, of the reusable module may access the operation data from the disposable module 220. As discussed herein, the operation data may include operation data and sensor life data.

At block 1456, the operation data is analyzed. The processor 254 of the reusable module 250 may perform the analysis. Optionally, the reusable module 250 may relay the operation data to the computing system 206, and the computing system 206 may perform the analysis of the operation data.

At block 1458, the disposable module 220 is identified based at least in part on the operation data and the analysis of the operation data. The identification of the disposable module 220 can include determination of a sensor type associated with the disposable module 220, determination of whether the sensor type associated with the disposable module corresponds to a configuration of the reusable module, determination of sensor life expectancy of a sensor associated with the disposable module, and the like.

### Communication with Other Sensor Devices

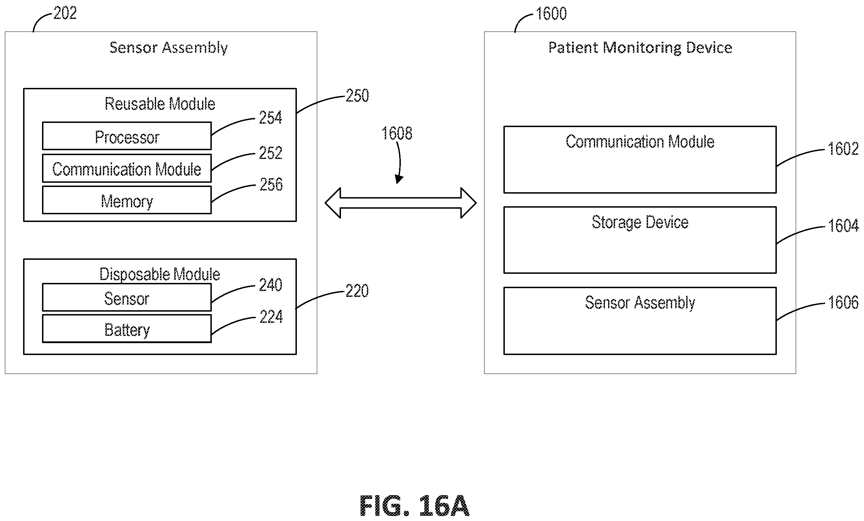

In some implementations, the sensor assembly 202 can communicate with other monitoring devices, such as a patient monitoring device 1600. Figure 16A illustrates a block diagram of the sensor assembly 202 in wireless communication with the patient monitoring device 1600. The patient monitoring device 1600 can include a communication module 1602, a storage device 1604, and a sensor assembly 1606. The patient monitoring device 1600 can be an activity tracker, a bedside monitor, a handheld monitor, and a wearable device. For example, the patient monitoring devices 1600 can be an electrocardiogram (ECG), thermometer, Radical (a patient monitoring device available at Masimo Corporation, Irvine, CA), Rad (a patient monitoring device available at Masimo Corporation, Irvine, CA), Root (a patient monitoring and connectivity platform available at Masimo Corporation, Irvine, CA), and the like. The patient monitoring device 1600 may collect, analyze, or display data related to various types of physiological parameters including, electrocardiogram, pulse rate, respiratory rate, body temperature, blood oxygen saturation (SpO₂), perfusion index (PI), Pleth Variability Index (PVi^{®}), total hemoglobin (SpHb^{®}), oxygen content (SpOC^{™}), methemoglobin (SpMet^{®}), Carboxyhemoglobin (SpCO^{®}), acoustic respiratory rate (RRa^{®}), electroencephalogram (EEG), enhanced Patient State Index (PSi), density spectral array (DSA), and the like.

The communication module 1602 can establish wired or wireless communication with various devices, networks, and the like. The sensor assembly 1606 can collect data, for example, associated with or related to a physiological condition of a patient. The sensor assembly 1606 may be directly in contact with the patient. The storage device 1604 may store data collected by the sensor assembly 1606.

The sensor assembly 1606 may include one or more sensors that can collect one or more types of physiological data described herein. For example, the patient monitoring device 1600 may be a holter monitor and the sensor assembly 1606 may include one or more leads that can be attached to, for example, a torso area of a patient. In one example, the sensor assembly 1606 includes three (3) leads. In another example, the sensor assembly 1606 includes twelve (12) leads.

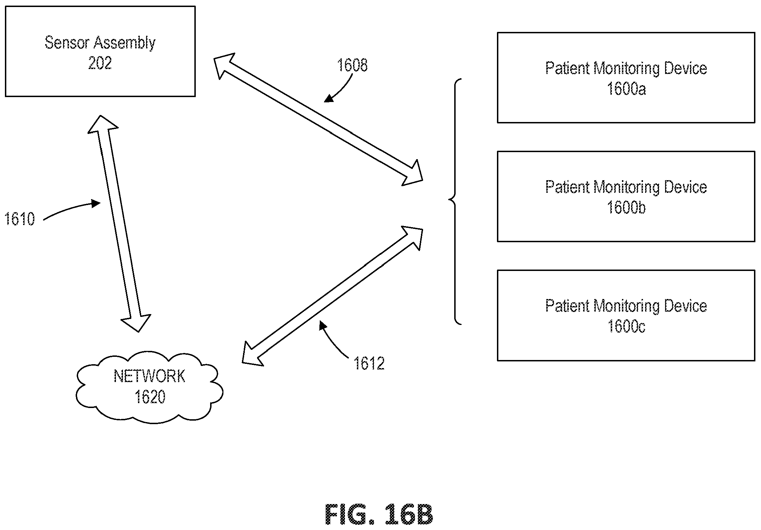

In some implementations, the sensor assembly 202 can communicate with the patient monitoring device 1600. The sensor assembly 202 may directly communicate with the patient monitoring device 1600 by establishing a direct communication with the patient monitoring device 1600. Alternatively and/or optionally, the sensor assembly 202 may indirectly communicate with the patient monitoring device 1600, for example, via a network 1620 (see Figure 16B) or a server.

As described herein, the sensor assembly 202 may store collected patient physiological data in the memory 256 of the reusable module 250 when the sensor assembly 202 is unable to transmit the data to, for example, the computing system 206 via, for example, a wireless communication. The sensor assembly 202, upon determining that it is unable to transmit collected data to, for example, the computing system 206 (for example, because of interrupted wireless communication between the sensor assembly 202 and the computing device 206), the sensor assembly 202 may alternatively, transmit the collected patient physiological data to the patient monitoring device 1600 via wireless communication 1608. The patient monitoring device 1600 may receive the patient physiological data via the communication module 1602 and store the data. The data may be stored in the storage device 1604.

The patient monitoring device 1600 may transmit the patient physiological data back to the sensor assembly 202 when the communication between the sensor assembly 202 and the computing system 206 is restored. In some implementations, the sensor assembly 202 generates and transmits a notification to the patient monitoring device 1600 indicating that the communication between the sensor assembly 202 and the computing system 206 is restored. Upon receiving the notification from the sensor assembly 202, the patient monitoring device 1600 can transmit the stored patient physiological data (that is, the patient physiological data that the sensor assembly 202 transmitted to the patient monitoring device 1600 upon determining that it is unable to transmit collected data to, for example, the computing system 206) to the sensor assembly 202 via the wireless communication 1608. The sensor assembly 202, upon receiving the patient physiological data from the patient monitoring device 1600, can transmit the data to the computing device 206. As such, the sensor assembly 202 can use the patient monitoring device 1600 as a backup data storage device.

In some implementations, the sensor assembly 202 can communicate with one or more patient monitoring devices 1600. Figure 16B illustrates a block diagram showing the sensor assembly 202 in wireless communication with the patient monitoring devices 1600a, 1600b, 1600c, and the network 1620. The sensor assembly 202 may establish with one or more of the patient monitoring devices 1600a, 1600b, 1600c, at any time. In some implementations, the sensor assembly 202, to preserve power stored in the battery 224 of the disposable module 220, may, by default, not establish wireless communication when it is able to communicate with, for example, the computing system 206. As such, the sensor assembly 202 may establish wireless communication with one or more of the patient monitoring devices 1600a, 1600b, 1600c, when it determines that a wireless communication with the sensor assembly 202 is no longer available and thus, for example, unable transmit patient physiological data to the computing device 206.

In some implementations, the sensor assembly 202 may be able to communicate with the network 1620 via wireless communication 1610. As noted herein, the network 1620 may be in communication with the computing system 206. Accordingly, even if the sensor assembly 202 is unable to establish communication with the computing system 206 directly, it nevertheless may be able to indirectly communicate with the computing system 206 via the network 1620.

In some implementations, the patient monitoring device 1600 may communicate with the network 1620 via wireless communication 1612. For example, the patient monitoring devices 1600a, 1600b, 1600c, upon receiving patient physiological data from the sensor assembly 202, may transmit the data to the network 1620 via wireless communication 1612. The network 1620 may be a server in communication with the computing device 206. Upon receiving the patient physiological data from the patient monitoring devices 1600a, 1600b, 1600c, the network 1620 can transmit the data to the computing device 206 for, for example, analysis of the data, determination of physiological parameters based on the data, and generate a display of the physiological parameters, for example, on a display. In some implementations, the transmission of the patient physiological data between the sensor assembly 202, the patient monitoring devices 1600a, 1600b, 1600c, and the network 1620 may occur with or without delay. For example, the patient monitoring devices 1600a, 1600b, 1600c may transmit the patient physiological data to the network 1620 immediately after it receives the patient physiological data from the sensor assembly 202. Alternatively, the patient monitoring devices 1600a, 1600b, 1600c may transmit the patient physiological data to the network 1620 after a predetermined time period is elapsed.

In some implementations, the sensor assembly 202 generates a packet that includes the patient physiological data and instructions for the patient monitoring devices 1600a, 1600b, 1600c. The instructions can cause the patient monitoring devices 1600a, 1600b, 1600c to transmit the patient physiological data to, for example, the computing device 206 or the network 1620 after receipt. In another example, the instructions can cause the patient monitoring devices 1600a, 1600b, 1600c to store the patient physiological data until the patient monitoring devices 1600a, 1600b, 1600c are connected to, for example, the computing system 206 or the network 1620. In yet another example, the instructions can cause the patient monitoring devices 1600a, 1600b, 1600c to store the patient physiological data.

In some implementations, the transmission of the patient physiological data between the sensor assembly 202, the patient monitoring devices 1600a, 1600b, 1600c, and the network 1620 may occur if a predetermined condition is met. For example, the sensor assembly 202 (or the reusable module 250 of the sensor assembly 202) may transmit collected patient physiological data to one or more of the patient monitoring devices 1600a, 1600b, 1600c when it is unable to establish wireless communication with, for example, the computing device 206 or the network 1620 for a predetermined amount of time. The predetermined amount of time (for example, a timer) may be ten (10) seconds, thirty (30) seconds, one (1) minute, two (2) minutes, five (5) minutes, ten (10) minutes, or any duration sufficient to prevent or reduce data loss or unnecessary power consumption from unsuccessful attempts to establish wireless communication.

In another example, the sensor assembly 202 (or the reusable module 250 of the sensor assembly 202) may transmit collected patient physiological data to one or more of the patient monitoring devices 1600a, 1600b, 1600c after a predetermined number of unsuccessful attempts to establish wireless communication with, for example, the computing device 206 or the network 1620. The predetermined number of unsuccessful attempts may be one (1), two (2), five (5), ten (10), twenty (20), or any number sufficient to prevent or reduce data loss or unnecessary power consumption from unsuccessful attempts to establish wireless communication.

The predetermined condition may be modifiable and may be modified by a user (for example, a patient) or a care provider (for example, a doctor). Alternatively, a user (for example, care provider such as a nurse or a doctor) input or request may cause or allow transmissions of patient physiological data between the sensor assembly 202, the patient monitoring devices 1600a, 1600b, 1600c, and the network 1620. For example, a nurse may determine that the sensor assembly 202 is no longer communicating with the computing system 206 and provide a user input to the sensor assembly 202 to allow the sensor assembly 202 to transmit patient physiological data to the patient monitoring devices 1600a, 1600b, 1600c.

In some implementations, a priority scheme may be used between the sensor assembly 202 and the patient monitoring devices 1600a, 1600b, 1600c. For example, the sensor assembly 202 may generate and transmit a request to the patient monitoring devices 1600a, 1600b, 1600c for connectivity information associated with the wireless communication 1612 between the patient monitoring devices 1600a, 1600b, 1600c and the network 1620. Upon receipt of the request for the connectivity information, the patient monitoring devices 1600a, 1600b, 1600c may, in response, transmit the connectivity information back to the sensor assembly 202. The connectivity information may be associated with, for example, connectivity strength between the patient monitoring devices 1600a, 1600b, 1600c and the network 1620.

Based on the received connectivity information, the sensor assembly 202 can identify a patient monitoring device (for example, patient monitoring device 1600c) for transmitting patient physiological data. The sensor assembly 202 can transmit patient physiological data to the identified patient monitoring device, and the identified patient monitoring device can relay the data to the network 1620 as discussed herein.

In another example, the priority scheme may be related to battery status and/or storage device status. For example, the sensor assembly 202 may generate and transmit a request to the patient monitoring devices 1600a, 1600b, 1600c for battery status and/or storage device status information associated with the patient monitoring devices 1600a, 1600b, 1600c. Upon receipt of the request for the battery status and/or storage device status information, the patient monitoring devices 1600a, 1600b, 1600c may, in response, transmit the battery status and/or storage device status information back to the sensor assembly 202. The battery status information may be related to (1) a charge level of battery, (2) power usage of the patient monitoring device 1600a, 1600b, 1600c, and (3) expected amount of power usage from data transmission between the sensor assembly 202 and the patient monitoring device 1600. The storage device status may be related to (1) an amount of data storage available, (2) expected amount of data from the sensor assembly 1606, and (3) expected amount of data from the sensor assembly 202.

Based on the received battery status and/or storage device status information, the sensor assembly 202 can identify a patient monitoring device (for example, patient monitoring device 1600c) for transmitting patient physiological data. The sensor assembly 202 can transmit patient physiological data to the identified patient monitoring device, and the identified patient monitoring device can store the patient physiological data.

The use of the patient monitoring devices 1600a, 1600b, 1600c may allow increased flexibility for handling, storage, and transmission of patient physiological data to the computing device 206. For example, the patient monitoring devices 1600a, 1600b, 1600c may have longer battery life and/or larger data storage capacity than the sensor assembly 202. By transmitting patient physiological data to the patient monitoring devices the patient monitoring devices 1600a, 1600b, 1600c for, for example, storage until wireless communication between the sensor assembly 202 and the computing system 206 is restored may allow recovery of greater amount of data. Moreover, the use of the patient monitoring devices 1600a, 1600b, 1600c may provide increased flexibility for data transmission as they provide additional avenues for transmission of data (for example, patient physiological data) between the sensor assembly 202 and the computing system 206 via the wireless communication 1608 between the sensor assembly 202 and the patient monitoring devices 1600a, 1600b, 1600c, and the wireless communication 1612 between the patient monitoring devices 1600a, 1600b, 1600c and the network 1620.

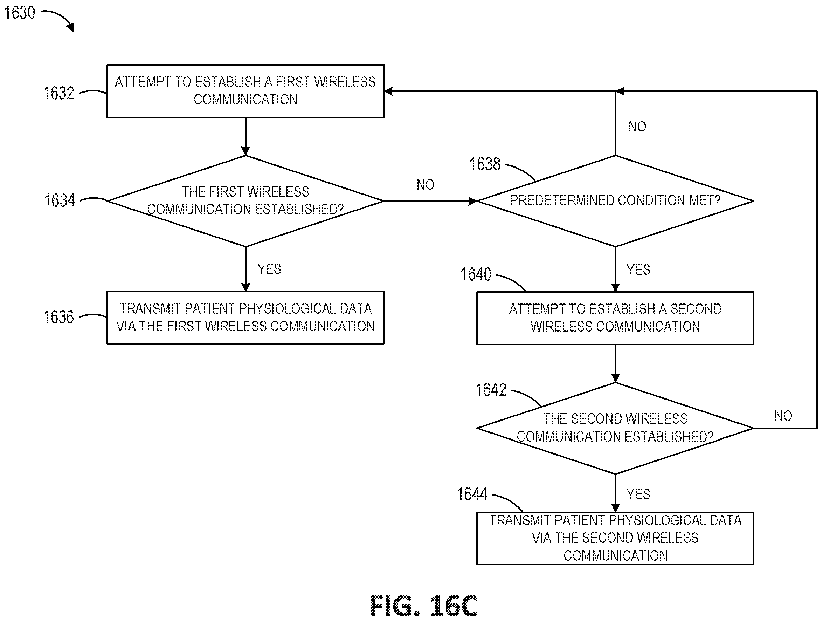

Figure 16C illustrates an example method 1630 for transmitting patient physiological data by the sensor assembly 202. The method 1630 may be performed by the sensor assembly 202 (or processor 254 of the reusable module 250) or any device in communication with the sensor assembly 202. At block 1632, the sensor assembly 202 attempts to establish a first wireless communication. The first wireless communication can be between the sensor assembly 202 and, for example, the computing system 206 or the network 1620.

At block 1634, the sensor assembly 202 determines whether the first wireless communication is established, for example, between the sensor assembly 202 and the computing system 206 or the network 1620. If the sensor assembly 202 determines that the first wireless communication is established, then the sensor assembly 202 transmits patient physiological data to, for example, the computing system 206 or the network 1620 via the first wireless communication at block 1636. If the sensor assembly 202 determines that the first wireless communication is not established, the sensor assembly 202 determines whether a predetermined condition is satisfied. As discussed herein, the predetermined condition can include, but not limited to, whether the sensor assembly 202 made a predetermined number of unsuccessful attempts of establishing the first wireless communication with, for example, the computing system 206 or the network 1620 or whether a predetermined amount of time has elapsed since the sensor assembly 202 failed to establish the first wireless communication with, for example, the computing system 206 or the network 1620.

If the sensor assembly 202 determines that the predetermined condition is not satisfied, the sensor assembly 202 attempts to establish the first wireless communication at block 1632. Alternatively, if the sensor assembly 202 determines that the predetermined condition is satisfied, then the sensor assembly 202 attempts to establish a second wireless communication with, for example, the patient monitoring devices 1600a, 1600b, 1600c at block 1640. At block 1642, the sensor assembly 202 determines whether the second wireless communication is established. If the second communication is established, then the sensor assembly 202 transmits patient physiological data to, for example, the patient monitoring devices 1600a, 1600b, 1600c via the second wireless communication. However, if the second wireless communication is not established, then the sensor assembly 202 attempts to establish the first wireless communication at block 1632. Optionally, upon determining that the second wireless communication is not established, then the sensor assembly 202, instead of attempting to establish the first wireless communication, may store the patient physiological data in the memory 256.

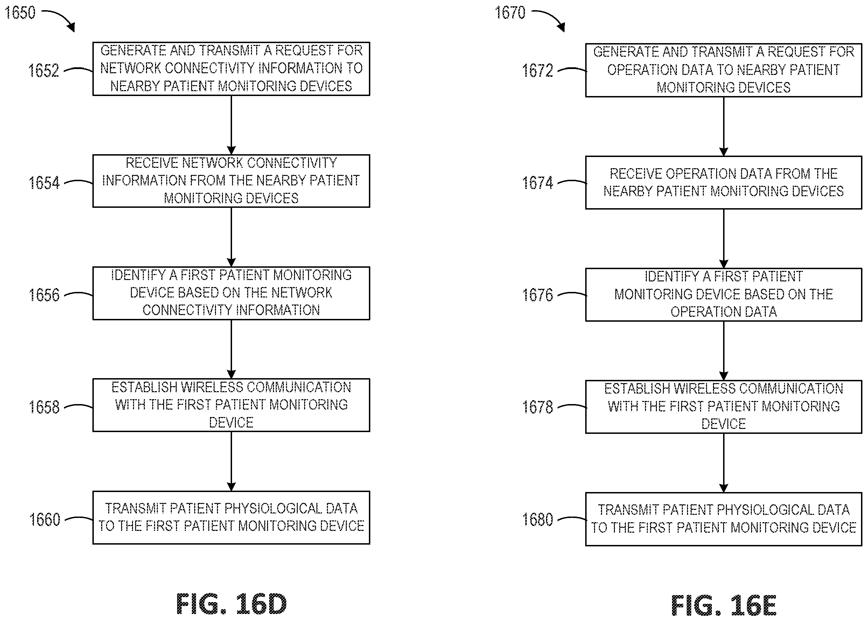

Figure 16D illustrates an example method 1650 for transmitting patient physiological data to a patient monitoring device. At block 1652, the sensor assembly 202 transmits a request for network connectivity information to one or more patient monitoring devices (for example, the patient monitoring devices 1600a, 1600b, 1600c). The one or more patient monitoring devices may be proximate to the sensor assembly 202. The one or more patient monitoring devices may or may not be coupled to a patient.

At block 1654, the sensor assembly 202 receives requested network connectivity information from the one or more patient monitoring devices. As discussed herein, the network connectivity information may be associated with, for example, network connectivity strength between the one or more patient monitoring devices and, for example, the network 1620. At block 1656, the sensor assembly 202 identifies a first patient monitoring device based on the network connectivity information. For example, in order to ensure uninterrupted, reliable, yet fast wireless transmission of patient physiological data, the sensor assembly 202 may identify a patient monitoring device that has the strongest, most reliable network connection with, for example, the network 1620 or the computing device 206.

At block 1658, the sensor assembly 202 (or the processor 254 of the reusable module 250) may establish a wireless communication with the first patient monitoring device (that is, one identified at block 1656). At block 1660, the sensor assembly 202 may transmit patient physiological data to the first patient monitoring device. As discussed herein, the first patient monitoring device may subsequently transmit the data to the network 1620.

Figure 16E illustrates an example method 1670 for transmitting patient physiological data to a patient monitoring device. At block 1672, the sensor assembly 202 transmits a request for operation data to one or more patient monitoring devices (for example, the patient monitoring devices 1600a, 1600b, 1600c). The one or more patient monitoring devices may or may not be proximate to the sensor assembly 202. The one or more patient monitoring devices may or may not be coupled to a patient.

At block 1674, the sensor assembly 202 receives requested operation data information form the one or more patient monitoring devices. As discussed herein, the operation data may be associated with, for example, (1) a battery charge level, (2) power consumption level, and (3) expected amount of power usage from data transmission. At block 1676, the sensor assembly 202 identifies a first patient monitoring device based on the battery status information. For example, in order to maximize the amount of patient physiological data stored, the sensor assembly 202 may identify a patient monitoring device that has the highest battery charge, lowest power usage, and/or least expected amount of power usage from data transmission.

At block 1678, the sensor assembly 202 (or the processor 254 of the reusable module 250) may establish a wireless communication with the first patient monitoring device (that is, one identified at block 1676). At block 1680, the sensor assembly 202 may transmit patient physiological data to the first patient monitoring device. As discussed herein, the first patient monitoring device may subsequently transmit the data to the network 1620.

### Data Transmission to Care Providers

In some implementations, the communication module 252 of the reusable module 250 of the sensor assembly 202 is optional and the reusable module 250 may not include the communication module 252. As such, the reusable module 250 may not establish wireless communication with nearby devices, routers, access points, and the like, and may not transmit patient physiological data to, for example, the computing system 206 or the network 1620. Without the communication module 252, the reusable module 250 may receive physiological data from the sensor 240 of the disposable module 220 and store the data in, for example, the memory 256. Such a configuration can include a significantly larger memory to allow the communication module 252 to store high fidelity data for multiple days, for example three to seven days of continuous monitoring. This configuration can advantageously allow the reusable module 250 to operate for a longer period of time by reducing the amount of power consumption by storing patient physiological data in the memory 256 instead of wirelessly transmitting the data to, for example, the computing system 206, via the communication module 252.

In some implementations, the reusable module 250 includes the communication module 252 that can be disabled. For example, care providers or patients themselves may be able to disable functionality of the communication module 252 to prevent wireless transmission of patient physiological data from the sensor assembly 202 to, for example, the computing system 206 or the network 1620. As discussed herein, such wireless transmission of patient physiological data may be transmitted via wireless communication protocols including, but not limited to, Wi-Fi, ZigBee, Lo-Fi, Bluetooth^{®}, Zwave, MiWI, near-field communication (NFC), and the like. In some implementations, the functionality of the communication module 252 can be enabled or disabled remotely.

Optionally, the reusable module 250 (or processor 254 of the reusable module 250) may generate and provide a notification upon determining that the storage capacity of the memory 256 satisfies a predetermined condition. For example, the predetermined condition may be a percentage of storage available in the memory 256 (e.g., less than or equal to 10% of available storage). In another example, the predetermined condition may be an estimated duration of time during which the sensor assembly 202 can collect and store data in the memory 256 (e.g., less than or equal to one (1) day's worth of patient physiological data).

The notification may be provided to, for example, a patient using the sensor assembly 202 (for example, at his or her home) or to a care provider having access to the sensor assembly 202. The sensor assembly 202 (for example, the reusable module 250 or the disposable module 220) can have an LED or a display that can display the notification about the storage capacity of the memory 256. Additionally and/or alternatively, the sensor assembly 202 (or the processor 254 of the reusable module 250) can, for example, transmit the notification to a care provider's computing device via, for example, the network 1620 or the computing system 206. The notification can include a message that will prompt the patient or the care provider that the memory 256, for example, is almost full. The notification can advantageously allow a patient using the sensor assembly 202 to, for example, send the reusable module 250 with stored patient physiological data to a care provider and/or request another reusable module 250, or allow a care provider to identify a patient with a reusable module 250 that needs to be replaced. Once a care provider identifies a patient with a reusable module 250 that needs to be replaced, the care provider can contact the patient to come into a care facility, for example, a hospital, to drop off the reusable module 250 and pick up a new reusable module 250, or send the reusable module 250 that needs to be replaced, for example, to a care facility via mail.

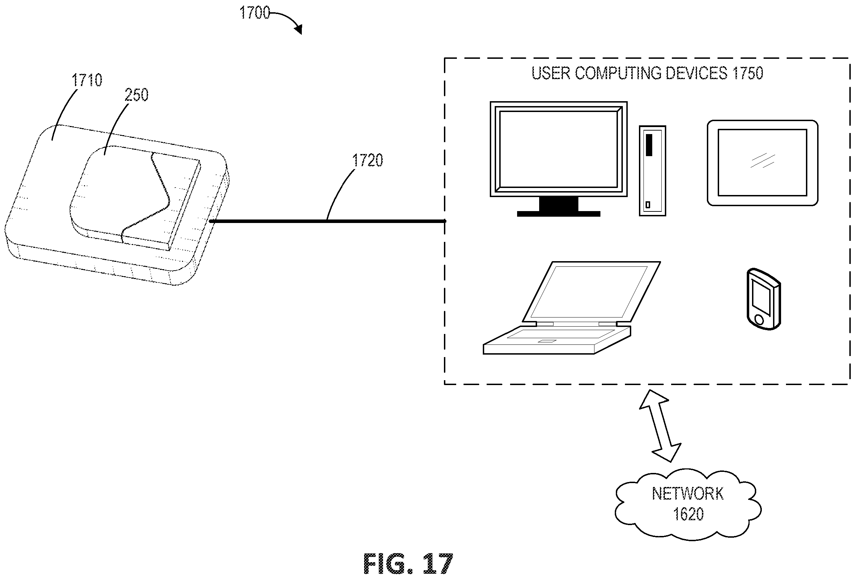

Figure 17 illustrates an example environment 1700 between the reusable module 250 and user computing devices 1750. In the example shown in Figure 17, the reusable module 250 can be connected the user computing devices 1750 via a terminal 1710 and a cable 1720. The user computing devices 1750 can include a desktop computer, a tablet, a laptop computer, a mobile communication device, and the like.

The cable 1720 can allow transmission of data between the terminal 1710 and the user computing devices 1750. In some implementations, the cable 1720 is optional and the terminal 1710 can wirelessly communicate with the user computing devices 1750.

The terminal 1710 can receive and establish communication with the reusable module 250. In some implementations, the terminal 1710 can detect communication with the reusable module 250 and initiate transfer of patient physiological data (that is, patient physiological data stored in the memory 256 of the reusable module 250) from the reusable module 250 to the terminal 1710. Alternatively, the reusable module 250 (or the processor 254 of the reusable module 250) can detect communication with the terminal 1710 and initiate transfer of patient physiological data (that is, patient physiological data stored in the memory 256 of the reusable module 250) from the reusable module 250 to the terminal 1710.

In some implementations, a user input may cause or allow the data transfer between the reusable module 250 and the terminal 1710. For example, the terminal 1710 detects the connection with the reusable module 250 and sends a notification to the user computing devices 1750. The notification can include a request for data transfer between the reusable module 250 and the terminal 1710 (and/or the user computing devices 1750) and prompt a user input to allow or disallow the data transfer. If the user provides a user input allowing the data transfer, the data transfer between the reusable module 250 and the terminal 1710 can occur. If the user provides a user input disallowing the data transfer, the data transfer between the reusable module 250 and the terminal 1710 may not occur. Once the terminal 1710 receives patient physiological data from the reusable module 250, it can relay the data to the user computing devices 1750.

As shown in Figure 17, the user computing devices 1750 can be connected to the network 1620. The user computing devices 1750 can transmit the data received from the reusable module 250 to the network 1620 for further analysis, processing, or storage of the data.

The user computing devices 1750 may be located remotely from a patient using the sensor assembly 202. As discussed herein, a patient may be located remotely from a care facility, for example, a hospital, for remote patient monitoring. Such remote monitoring of patients can be advantageous for reducing or preventing the likelihood of transmission of diseases such as COVID-19 between patients and care providers. In some implementations, the user computing devices can be a patient's desktop computer, a tablet, a laptop computer, or a mobile communication device, for example, a smartphone. Accordingly, the terminal 1710 can be located proximate to the patient's, for example, desktop computer. The terminal 1710 can facilitate transfer of patient physiological data between the sensor assembly 202 and the patient's desktop computer, as discussed herein, and the patient's desktop computer can subsequently transmit the data to the network 1620. Such setting can advantageously prevent delays caused by, for example, patients shipping their sensor systems 202 to their care providers or patients visiting his or her care provider to drop off their sensor systems 202. Moreover, it can further reduce the likelihood of transmission of diseases, for example COVID-19, by allowing the data transmission to occur at the patient's home and thereby preventing possibly-contaminated reusable modules 250 from being shipped to or entering care facilities (for example, hospitals).

### Terminology

Many other variations than those described herein will be apparent from this disclosure. For example, depending on the embodiment, certain acts, events, or functions of any of the algorithms described herein can be performed in a different sequence, can be added, merged, or left out altogether (e.g., not all described acts or events are necessary for the practice of the algorithms). Moreover, in certain embodiments, acts or events can be performed concurrently, e.g., through multi-threaded processing, interrupt processing, or multiple processors or processor cores or on other parallel architectures, rather than sequentially. In addition, different tasks or processes can be performed by different machines and/or computing systems that can function together.

The various illustrative logical blocks, modules, and algorithm steps described in connection with the embodiments disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative logical blocks and modules described in connection with the embodiments disclosed herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can include electrical circuitry configured to process computer-executable instructions. In another embodiment, a processor includes an FPGA or other programmable device that performs logic operations without processing computer-executable instructions. A processor can also be implemented as a combination of computing devices, e.g., a combination of a DSP and a microprocessor, a plurality of microprocessors, one or more microprocessors in conjunction with a DSP core, or any other such configuration. A computing environment can include any type of computer system, including, but not limited to, a computer system based on a microprocessor, a mainframe computer, a digital signal processor, a portable computing device, a device controller, or a computational engine within an appliance, to name a few.

The steps of a method, process, or algorithm described in connection with the embodiments disclosed herein can be embodied directly in hardware, in a software module stored in one or more memory devices and executed by one or more processors, or in a combination of the two. A software module can reside in RAM memory, flash memory, ROM memory, EPROM memory, EEPROM memory, registers, hard disk, a removable disk, a CD-ROM, or any other form of non-transitory computer-readable storage medium, media, or physical computer storage known in the art. An example storage medium can be coupled to the processor such that the processor can read information from, and write information to, the storage medium. In the alternative, the storage medium can be integral to the processor. The storage medium can be volatile or nonvolatile The processor and the storage medium can reside in an ASIC.

Conditional language used herein, such as, among others, "can," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

The invention is defined by the appended claims.

The term "and/or" herein has its broadest, least limiting meaning which is the disclosure includes A alone, B alone, both A and B together, or A or B alternatively, but does not require both A and B or require one of A or one of B. As used herein, the phrase "at least one of' A, B, "and" C should be construed to mean a logical A or B or C, using a non-exclusive logical or.

The apparatuses and methods described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

Although the foregoing disclosure has been described in terms of certain preferred embodiments, other embodiments will be apparent to those of ordinary skill in the art from the disclosure herein. Additionally, other combinations, omissions, substitutions and modifications will be apparent to the skilled artisan in view of the disclosure herein. Accordingly, the present invention is not intended to be limited by the description of the preferred embodiments, but is to be defined by reference to claims.

## Claims

1. A system (100) for collecting physiological data from a patient (110), the system (100) comprising:
a reusable module (250) comprising:
a processor (254);
a first memory (256); and
a wireless communication module configured to establish a wireless communication (204) with a patient monitoring system (100); and
a disposable module (220) comprising:
a sensor element (240) configured to collect physiological data from a patient (110);
a second memory (256) configured to store operation data associated with the sensor element (240); and
a battery (224), and wherein
the disposable module (220) is validated based at least in part on the operation data;
the first memory (256) is configured to store the physiological data collected by the sensor element (240) of the disposable module (220);
sensor life expectancy is determined based at least in part on the operation data and sensor life data, and wherein the sensor life expectancy represents the expected operation time of the disposable module (220);
the sensor life expectancy is automatically updated when there is a change in patient condition or a change in operation condition for the disposable module (220); and
detecting the change in patient condition triggers the collecting of the physiological data at a higher fidelity causing increased power consumption by the disposable module.

2. The system (100) of the previous claim, wherein the operation data comprises sensor type information associated with the disposable module (220).

3. The system (100) of the previous claim, wherein the sensor type information indicates one or more types of sensors associated with the disposable module (220).

4. The system (100) of any of the previous claims, wherein the reusable module (250) assembly is associated with a sensor type, and wherein the disposable module (220) is validated based at least in part on a comparison between the sensor type associated with the reusable module (250) assembly and the sensor type information associated with the disposable module (220).

5. The system (100) of any of the previous claims, wherein the sensor life data comprises sensor use information and one or more functions, and wherein sensor life data is stored in the memory of the disposable module (220).

6. The system (100) of any of the previous claims, wherein the first memory (256) stores a default length of time, and wherein the first memory (256) is configured to store the physiological data for the default length of time prior to the wireless communication module establishing the wireless communication when the length of time is not provided.

7. The system (100) of any of the previous claims, wherein the physiological data is stored when irregularities are sensed.

8. The system (100) of the previous claim, wherein the irregularities include at least one of: low blood pressure readings, high blood pressure readings, low respiratory rate readings, high respiratory rate readings, blood oxygen desaturations, irregular heartbeats, consistently low or declining blood oxygen saturation readings, low heart rates, or high heart rates.

9. The system (100) of any of the previous claims, wherein the processor (254) of the reusable module (250) is configured to transmit the stored physiological data to a local or a remote storage via the wireless communication module when a wireless communication between the wireless communication module and an online server is established.

10. The system (100) of any of the previous claims, wherein the physiological data collected by the sensor element (240) has high fidelity.

11. The system (100) of any of the previous claims, wherein the physiological data collected by the sensor element (240) has low fidelity.

12. The system (100) of any of the previous claims, wherein fidelity of the physiological data collected by the sensor element (240) varies.

13. The system (100) of the previous claim, wherein the fidelity of the physiological data collected by the sensor element (240) varies based at least in part on a length of time specified for storing the physiological data within the first memory (256).

14. The system (100) of claim 12, wherein the fidelity of the stored physiological data collected by the sensor element (240) varies based at least in part a type of physiological data or a type of health-related events.

15. The system (100) of any of the previous claims, wherein the first memory (256) stores the physiological data collected by the sensor element (240) from time reusable module (250) is attached to the disposable module (220) until time the reusable portion is detached from the disposable module (220) or the battery (224) of the disposable module (220) fails.

## Patentansprüche

1. System (100) zum Erfassen physiologischer Daten von einem Patienten (110), wobei das System (100) aufweist:
ein wiederverwendbares Modul (250), das aufweist:
einen Prozessor (254);
einen ersten Speicher (256); und
ein Drahtloskommunikationsmodul, das dafür konfiguriert ist, eine Drahtloskommunikation (204) mit einem Patientenüberwachungssystem (100) herzustellen; und
ein Einwegmodul (220), das aufweist:
ein Sensorelement (240), das dafür konfiguriert ist, physiologische Daten von einem Patienten (110) zu erfassen;
einen zweiten Speicher (256), der dafür konfiguriert ist, dem Sensorelement (240) zugeordnete Betriebsdaten zu speichern; und
eine Batterie (224), und wobei
das Einwegmodul (220) zumindest teilweise basierend auf den Betriebsdaten validiert wird,
der erste Speicher (256) dafür konfiguriert ist, die durch das Sensorelement (240) des Einwegmoduls (220) erfassten physiologischen Daten zu speichern,
die Sensorlebensdauer zumindest teilweise basierend auf den Betriebsdaten und den Sensorlebensdauerdaten bestimmt wird, und wobei die Sensorlebensdauer die erwartete Betriebszeit des Einwegmoduls (220) darstellt,
die Sensorlebensdauer automatisch aktualisiert wird, wenn eine Änderung des Patientenzustands oder eine Änderung des Betriebszustands des Einwegmoduls (220) auftritt, und
das Erfassen der Änderung des Patientenzustands das Erfassen der physiologischen Daten mit einer höheren Genauigkeit triggert, was einen erhöhten Stromverbrauch durch das Einwegmodul verursacht.

2. System (100) nach Anspruch 1, wobei die Betriebsdaten dem Einwegmodul (220) zugeordnete Sensortypinformation aufweisen.

3. System (100) nach dem vorhergehenden Anspruch, wobei die Sensortypinformation einen oder mehrere Sensortypen anzeigt, die dem Einwegmodul (220) zugeordnet sind.

4. System (100) nach einem der vorhergehenden Ansprüche, wobei das wiederverwendbare Modul (250) einem Sensortyp zugeordnet ist, und wobei das Einwegmodul (220) zumindest teilweise basierend auf einem Vergleich zwischen dem dem wiederverwendbaren Modul (250) zugeordneten Sensortyp und der dem Einwegmodul (220) zugeordneten Sensortypinformation validiert wird.

5. System (100) nach einem der vorhergehenden Ansprüche, wobei die Sensorlebensdauerdaten Sensornutzungsinformation und eine oder mehrere Funktionen aufweisen, und wobei die Sensorlebensdauerdaten im Speicher des Einwegmoduls (220) gespeichert sind.

6. System (100) nach einem der vorhergehenden Ansprüche, wobei der erste Speicher (256) eine Standardzeitdauer speichert, und wobei der erste Speicher (256) dafür konfiguriert ist, die physiologischen Daten für die Standardzeitdauer zu speichern, bevor das Drahtloskommunikationsmodul die Drahtloskommunikation herstellt, wenn die Zeitdauer nicht bereitgestellt wird.

7. System (100) nach einem der vorhergehenden Ansprüche, wobei die physiologischen Daten gespeichert werden, wenn Unregelmäßigkeiten erfasst werden.

8. System (100) nach dem vorhergehenden Anspruch, wobei die Unregelmäßigkeiten mindestens eines der folgenden Merkmale aufweisen: niedrige Blutdruckmesswerte, hohe Blutdruckmesswerte, niedrige Atemfrequenzmesswerte, hohe Atemfrequenzmesswerte, Blutsauerstoffentsättigungen, unregelmäßige Herzschläge, konstant niedrige oder abnehmende Blutsauerstoffsättigungsmesswerte, niedrige Herzfrequenzen oder hohe Herzfrequenzen.

9. System (100) nach einem der vorhergehenden Ansprüche, wobei der Prozessor (254) des wiederverwendbaren Moduls (250) dafür konfiguriert ist, die gespeicherten physiologischen Daten über das Drahtloskommunikationsmodul an einen lokalen oder einen abgesetzten Speicher zu übertragen, wenn eine Drahtloskommunikation zwischen dem Drahtloskommunikationsmodul und einem Online-Server eingerichtet ist.

10. System (100) nach einem der vorhergehenden Ansprüche, wobei die durch das Sensorelement (240) erfassten physiologischen Daten eine hohe Genauigkeit aufweisen.

11. System (100) nach einem der vorhergehenden Ansprüche, wobei die durch das Sensorelement (240) erfassten physiologischen Daten eine geringe Genauigkeit aufweisen.

12. System (100) nach einem der vorhergehenden Ansprüche, wobei die Genauigkeit der durch das Sensorelement (240) erfassten physiologischen Daten variiert.

13. System (100) nach dem vorhergehenden Anspruch, wobei die Genauigkeit der durch das Sensorelement (240) erfassten physiologischen Daten zumindest teilweise basierend auf einer für das Speichern der physiologischen Daten im ersten Speicher (256) spezifizierten Zeitdauer variiert.

14. System (100) nach Anspruch 12, wobei die Genauigkeit der durch das Sensorelement (240) erfassten, gespeicherten physiologischen Daten zumindest teilweise basierend auf einem Typ physiologischer Daten oder einem Typ gesundheitsbezogener Ereignisse variiert.

15. System (100) nach einem der vorhergehenden Ansprüche, wobei der erste Speicher (256) die durch das Sensorelement (240) erfassten physiologischen Daten von dem Zeitpunkt, zu dem das wiederverwendbare Modul (250) am Einwegmodul (220) befestigt wird, bis zu dem Zeitpunkt speichert, zu dem der wiederverwendbare Abschnitt vom Einwegmodul (220) abgenommen wird oder die Batterie (224) des Einwegmoduls (220) ausfällt.

## Revendications

1. Système (100) destiné à recueillir des données physiologiques auprès d'un patient (110), le système (100) comprenant :
un module réutilisable (250) comprenant :
un processeur (254) ;
une première mémoire (256) ; et
un module de communication sans fil configuré pour établir une communication sans
fil (204) avec un système de surveillance de patient (100) ; et
un module jetable (220) comprenant :
un élément de capteur (240) configuré pour recueillir des données physiologiques auprès d'un patient (110) ;
une seconde mémoire (256) configurée pour stocker les données de fonctionnement associées à l'élément de capteur (240) ; et
une batterie (224), et dans lequel
le module jetable (220) est validé en se basant au moins en partie sur les données de fonctionnement ;
la première mémoire (256) est configurée pour stocker les données physiologiques recueillies par l'élément de capteur (240) du module jetable (220) ;
la durée de vie du capteur est déterminée en se basant au moins en partie sur les données de fonctionnement et les données de vie de capteur, et dans lequel la durée de vie du capteur représente le temps de fonctionnement attendu du module jetable (220) ;
la durée de vie du capteur est automatiquement mise à jour en cas de changement dans l'état du patient ou de changement dans l'état de fonctionnement du module jetable (220) ; et
la détection du changement dans l'état du patient déclenche le recueil des données physiologiques avec une fidélité plus élevée, ce qui entraîne une augmentation de la consommation d'énergie par le module jetable.

2. Système (100) selon la revendication précédente, dans lequel les données de fonctionnement comprennent des informations de type de capteur associées au module jetable (220).

3. Système (100) selon la revendication précédente, dans lequel les informations de type de capteur indiquent un ou plusieurs types de capteurs associés au module jetable (220).

4. Système (100) selon l'une quelconque des revendications précédentes, dans lequel l'ensemble module réutilisable (250) est associé à un type de capteur, et dans lequel le module jetable (220) est validé en se basant au moins en partie sur une comparaison entre le type de capteur associé à l'ensemble module réutilisable (250) et les informations de type de capteurs associées au module jetable (220).

5. Système (100) selon l'une quelconque des revendications précédentes, dans lequel les données de vie de capteur comprennent des informations d'utilisation de capteurs et une ou plusieurs fonctions, et dans lequel les données de vie de capteurs sont stockées dans la mémoire du module jetable (220).

6. Système (100) selon l'une quelconque des revendications précédentes, dans lequel la première mémoire (256) stocke une durée par défaut, et dans lequel la première mémoire (256) est configurée pour stocker les données physiologiques pour la durée par défaut avant que le module de communication sans fil n'établisse la communication sans fil lorsque la durée n'est pas fournie.

7. Système (100) selon l'une quelconque des revendications précédentes, dans lequel les données physiologiques sont stockées lorsque des irrégularités sont détectées.

8. Système (100) selon la revendication précédente, dans lequel les irrégularités incluent au moins une parmi : des mesures de pression artérielle faible, des mesures de pression artérielle élevée, des mesures de rythme respiratoire faible, des mesures de rythme respiratoire élevé, des désaturations en oxygène sanguin, des rythmes cardiaques irréguliers, des mesures de saturation en oxygène sanguin systématiquement faible ou décroissante, des rythmes cardiaques faibles, ou des rythmes cardiaques élevés.

9. Système (100) selon l'une quelconque des revendications précédentes, dans lequel le processeur (254) du module réutilisable (250) est configuré pour transmettre les données physiologiques stockées à un stockage local ou distant par l'intermédiaire du module de communication sans fil lorsqu'une communication sans fil entre le module de communication sans fil et un serveur en ligne est établie.

10. Système (100) selon l'une quelconque des revendications précédentes, dans lequel les données physiologiques recueillies par l'élément de capteur (240) ont une fidélité élevée.

11. Système (100) selon l'une quelconque des revendications précédentes, dans lequel les données physiologiques recueillies par l'élément de capteur (240) ont une fidélité faible.

12. Système (100) selon l'une quelconque des revendications précédentes, dans lequel la fidélité des données physiologiques recueillies par l'élément de capteur (240) varie.

13. Système (100) selon la revendication précédente, dans lequel la fidélité des données physiologiques recueillies par l'élément de capteur (240) varie en se basant au moins en partie sur une durée spécifiée pour le stockage des données physiologiques au sein de la première mémoire (256).

14. Système (100) selon la revendication 12, dans lequel la fidélité des données physiologiques stockées recueillies par l'élément de capteur (240) varie en se basant au moins en partie sur un type de données physiologiques ou un type d'événements liés à la santé.

15. Système (100) selon l'une quelconque des revendications précédentes, dans lequel la première mémoire (256) stocke les données physiologiques recueillies par l'élément de capteur (240) à partir du moment où le module réutilisable (250) est fixé au module jetable (220) jusqu'au moment où la partie réutilisable est détachée du module jetable (220) ou où la batterie (224) du module j etable (220) subit une défaillance.
